(19) Europäisches Patentamt
European Patent Office
Office européen des brevets

(11) **EP 2 349 167 B1**

(12) **EUROPEAN PATENT SPECIFICATION**

(45) Date of publication and mention
of the grant of the patent:
**05.08.2015 Bulletin 2015/32**

(21) Application number: **09822332.4**

(22) Date of filing: **22.10.2009**

(51) Int Cl.:
**A61N 5/02** *(2006.01)*

(86) International application number:
**PCT/US2009/005772**

(87) International publication number:
**WO 2010/047818 (29.04.2010 Gazette 2010/17)**

(54) **SYSTEMS FOR THE NON-INVASIVE TREATMENT OF TISSUE USING MICROWAVE ENERGY**

SYSTEME ZUR NICHTINVASIVEN BEHANDLUNG VON GEWEBE MIT MIKROWELLENENERGIE

SYSTÈMES DE TRAITEMENT NON INVASIF DE TISSUS EN UTILISANT L'ÉNERGIE DE MICROONDES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.12.2008 PCT/US2008/001365**
**23.02.2009 US 208315 P**
**17.04.2009 PCT/US2009/000240**
**22.10.2008 US 196948 P**
**16.10.2009 US 279153 P**

(43) Date of publication of application:
**03.08.2011 Bulletin 2011/31**

(73) Proprietor: **Miramar Labs, Inc.**
**Sunnyvale, CA 94085 (US)**

(72) Inventors:
• **KIM, Steven**
**Los Altos**
**CA 94024 (US)**
• **FRANCIS, Daniel**
**Mountain View**
**CA 94040 (US)**
• **JOHNSON, Jessi E.**
**Sunnyvale, California 94086 (US)**
• **SALAMINI, Alexey**
**San Francisco**
**CA 94110 (US)**
• **SU, Ted**
**Sunnyvale, CA 94086 (US)**

• **CHUN, Dong Hoon**
**Sunnyvale CA 94087 (US)**
• **BEN-HAIM, Yoav**
**San Francisco**
**CA 94110 (US)**
• **LOEW, Christopher**
**Palo Alto**
**CA 94303 (US)**
• **KOPELOW, Leo**
**San Francisco, California 94114 (US)**
• **CHEW, Sunmi**
**San Jose**
**CA 95126 (US)**

(74) Representative: **Shanks, Andrew et al**
**Marks & Clerk LLP**
**Aurora**
**120 Bothwell Street**
**Glasgow G2 7JS (GB)**

(56) References cited:
EP-A2- 1 346 753      WO-A1-2007/108516
WO-A2-2006/117682    US-A- 5 097 846
US-A- 5 097 846      US-A1- 2001 050 083
US-A1- 2001 050 083  US-A1- 2006 112 698
US-A1- 2007 179 482  US-A1- 2007 237 620
US-A1- 2007 255 355  US-B1- 6 334 074
US-B1- 6 334 074

## Description

## Field of the Invention

The present application relates to methods, apparatuses, and systems for the non-invasive delivery of energy, including microwave energy. In particular, the present application relates to methods, apparatuses, and systems for non- invasively delivering energy, such as, e.g., microwave energy, to epidermal, dermal, and subdermal tissue of an individual to achieve various therapeutic and/or aesthetic results.

## Background of the Invention

It is known that energy-based therapies can be applied to tissue throughout the body to achieve numerous therapeutic and/or aesthetic results. There remains a continual need to improve on the effectiveness of these energy-based therapies and provide enhanced therapeutic results with minimal adverse side effects or discomfort.

US 2001/0050083 (Marchitto et al.) discloses a method of delivering substances for permeation through the skin of a subject, by energizing the stratum corneum in conjunction with various delivery means including gels and patches.

US 5,097,846 (Larsen) discloses a method and apparatus for therapeutic deep heating of musculoskeletal tissues which are characterized by an improved transducer that serves simultaneously to couple the power from the generator into the patient and to sense the therapeutic response for treatment control including a method of manufactured and testing of contact applicators for dielectric heating of musculoskeletal tissue.

US 2007/0179482 (Anderson) discloses devices and methods which generate reduced pressure to treat biological external tissue using at least one energy source are disclosed.

## Summary of the Invention

Systems and methods apply, in a non- invasive manner, energy to a targeted tissue region employing a controlled source of energy, an applicator, and an applicator-tissue interface carried by the applicator. The systems and methods can generate and apply energy in a controlled fashion to form a predefined pattern of lesions that provide therapeutic benefit, e.g., to moderate or interrupt function of the sweat glands in the underarm (axilla).

## Brief Description of the Drawings

Fig. 1 is a perspective view of a system for applying, in a non-invasive manner, forms of energy to body tissue to achieve desired therapeutic and/or aesthetic results comprising a console, an applicator, and an applicator-tissue interface.
Figs. 2 to 4 are side and rear perspective views of the console shown in Fig. 1.
Figs. 5 and 6 are perspective views of the applicator and applicator-tissue interface shown in Fig. 1, with Fig. 5 showing the applicator-tissue interface joined to the applicator for use and Fig. 6 showing the applicator-tissue interface detached from the applicator prior to or after use.
Fig. 7 is an exploded perspective view of the applicator shown in Figs. 5 and 6.
Fig. 8 is an assembled interior view of the applicator shown in Fig. 7.
Fig. 9 is an exploded perspective view of the waveguide antenna array, waveguide cradle, and cooling plate carried on-board the applicator shown in Fig. 7.
Fig. 10 is an assembled perspective view of the waveguide antenna array, waveguide cradle, and cooling plate shown in Fig. 9.
Fig. 11 is a bottom view, partially broken away, of the waveguide antenna array, waveguide cradle, and cooling plate shown in Fig. 10.
Fig. 12A is an exploded perspective view of the applicator-tissue interface shown in Fig. 6.
Fig. 12B is an assembled side section perspective view of the applicator-tissue interface shown in Fig. 12A.
Fig. 13 is an assembled, bottom perspective view of the applicator-tissue interface attached to the waveguide antenna array, waveguide cradle, and cooling plate of the applicator for use.
Figs. 14A and 14B are top and bottom plane views of a applicator-tissue interface with interior patterns along its interior that may impress a "hickey pattern" on the skin drawn into the chamber.
Fig. 15 is a schematic view of the system shown in Fig. 1.
Figs. 16A and 16B are views of the custom designed multi-function plug at one end of the special purpose cable assembly that couples the applicator to the console, as shown in Fig. 1.
Figs. 16C and 16D are views of the vacuum trap that couples the applicator-tissue interface to the console, as shown in Fig. 1.
Figs. 17 and 18 are schematic views of the circuitry of the forward and reverse power detectors that may be carried on-board the applicator shown in Fig. 1.
Fig. 19A is a perspective view of the LED Indicator Board carried on-board the applicator and its functionality.
Figs. 19B, C, D, and E are illustrative views of LED displays that the LED Indicator Board shown in Fig. 19A can present to the caregiver holding the applicator.
Fig. 20 is a simplified anatomic side section view of human skin.

Fig. 21A is a partially schematic side section view of the applicator and applicator-tissue interface placed into contact with human skin prior to application of vacuum to the tissue acquisition chamber.

Fig. 21B is a partially schematic side section view of the applicator and applicator-tissue interface placed into contact with human skin after application of vacuum to the tissue acquisition chamber to draw skin into the chamber for treatment.

Fig. 22A is a partially schematic side section view of the applicator and applicator-tissue interface placed into contact with human skin after application of vacuum to the tissue acquisition chamber to draw skin into the chamber for treatment, and after the application of energy through a single waveguide antenna.

Fig. 22B is a schematic view of a lesion formed after the application of energy through a single waveguide antenna as shown in Fig. 22A.

Fig. 23A is a partially schematic side section view of the applicator and applicator-tissue interface placed into contact with human skin after application of vacuum to the tissue acquisition chamber to draw skin into the chamber for treatment, and after the application of energy through adjacent waveguide antennas in a phase drive mode.

Fig. 23B is a schematic view of a lesion pattern formed after the successive application of energy through single and adjacent waveguide antennas as shown in Figs. 22A and 23A.

Figs. 24A and 24B are views of representative treatment templates for use in methods and procedures according to the present invention.

Fig. 25 are perspective views of packaging for the applicator and applicator-tissue interface shown in Fig. 1 in kits together with instructions for use.

Fig. 26 is a perspective view of the display screen shown in Fig. 1, showing a screen of a representative graphical user interface.

Figs. 27 to 31 are schematic views of the logic and control components of a representative graphical user interface, which includes step-by-step instructions for using the components of the system, with cross reference to representative graphical screen shots.

Figs. 32 to 59 are screen shots of a representative graphical user interface executed according to the logic shown in Figs. 27 to 31.

Description of the Preferred Embodiments

This Specification discloses various systems and methods for applying, in a non-invasive manner, forms of energy to body tissue to achieve desired therapeutic and/or aesthetic results. As described, the systems and methods are particularly well suited for treating the epidermal, dermal, and sub-dermal tissue of an individual to treat, e.g., skin conditions, aesthetic conditions, glandular structures, vascular structures, or hair follicles. For this reason, the systems and methods will be described in this context, and, in particular, in the context of the application of electromagnetic microwave energy to sweat glands to treat hyperhidrosis, or excessive seating.

Still, it should be appreciated that the disclosed systems and methods are applicable for use in applying, in a non-invasive manner, microwave or other forms of energy to treat other conditions elsewhere in the body. Further, although the disclosure contained in this Specification is detailed and exact to enable those skilled in the art to practice the invention, the physical embodiments disclosed are intended to exemplify representative embodiments that highlight the technical features of the invention. The technical features of the invention may be embodied in other specific structures. While the preferred embodiments have been described, the details may be changed without departing from the technical features of the invention as defined in the claims.

I. System Overview

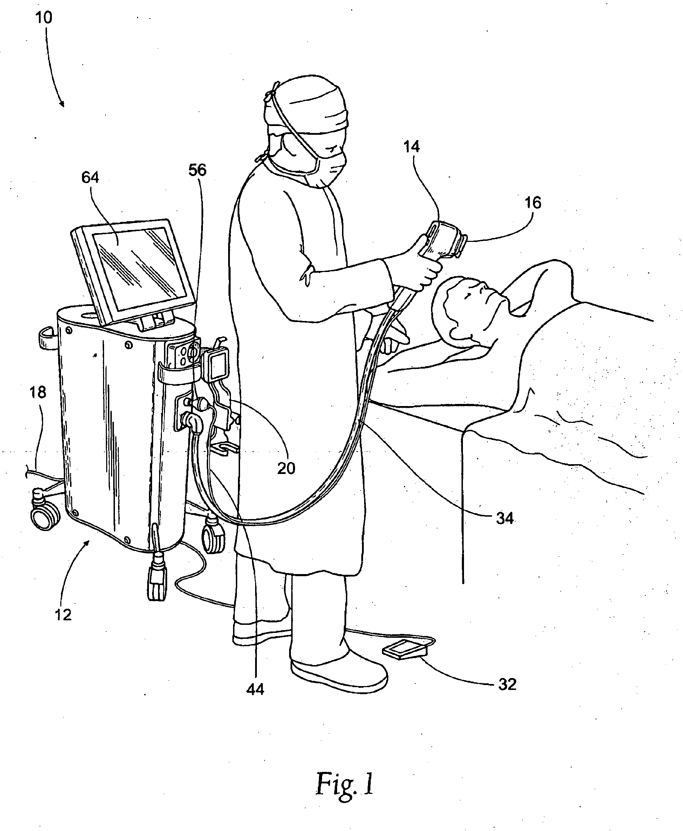

Fig. 1 shows a system 10 for applying, in a non-invasive manner, energy to a targeted tissue region that embodies the features of the invention. As shown in Fig. 1, the system 10 includes three main components. These are a system console 12; a system applicator 14; and an applicator-tissue interface 16 carried by the system applicator 14.

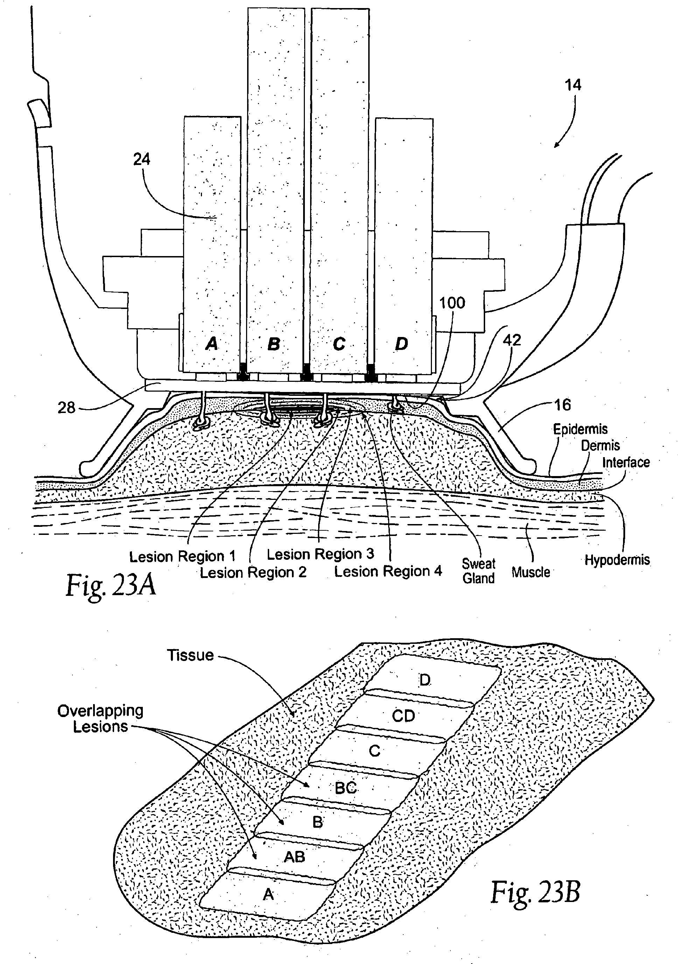

In the illustrative embodiment shown in Fig.I, and as will be described in further detail later, the system 10 is particularly sized and configured to generate and apply energy to the underarm (axilla) of an individual to form a predefined pattern of lesions (see, e.g., Fig. 23B). The pattern of lesions serves, e.g., to moderate or interrupt function of the sweat glands in the underarm. In this illustrative arrangement, the system 10 and its method of use can serve to treat, e.g., axillary hyperhidrosis or underarm sweating/odor.

A. The System Console

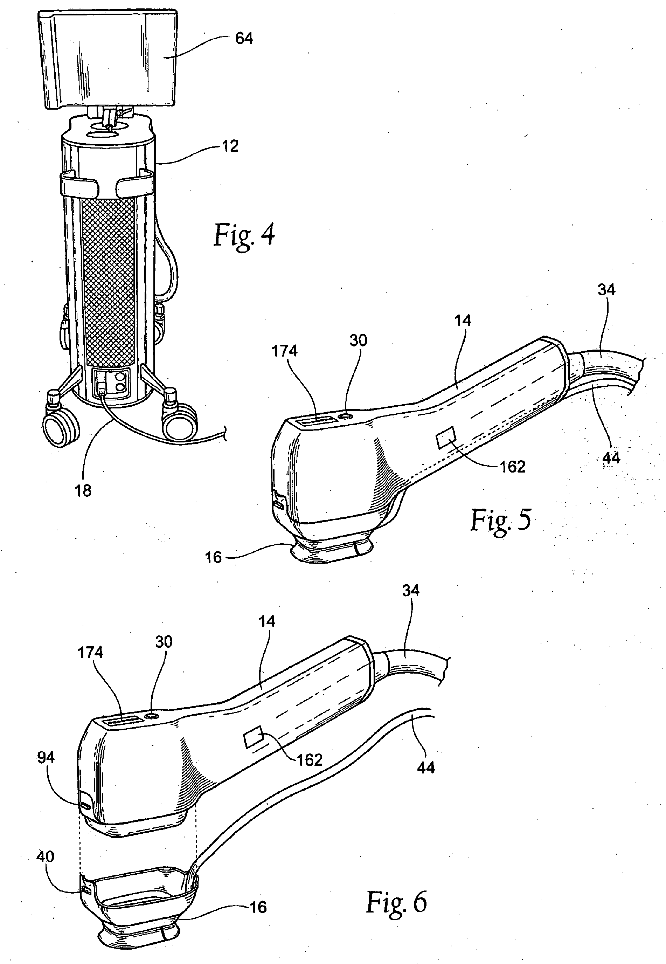

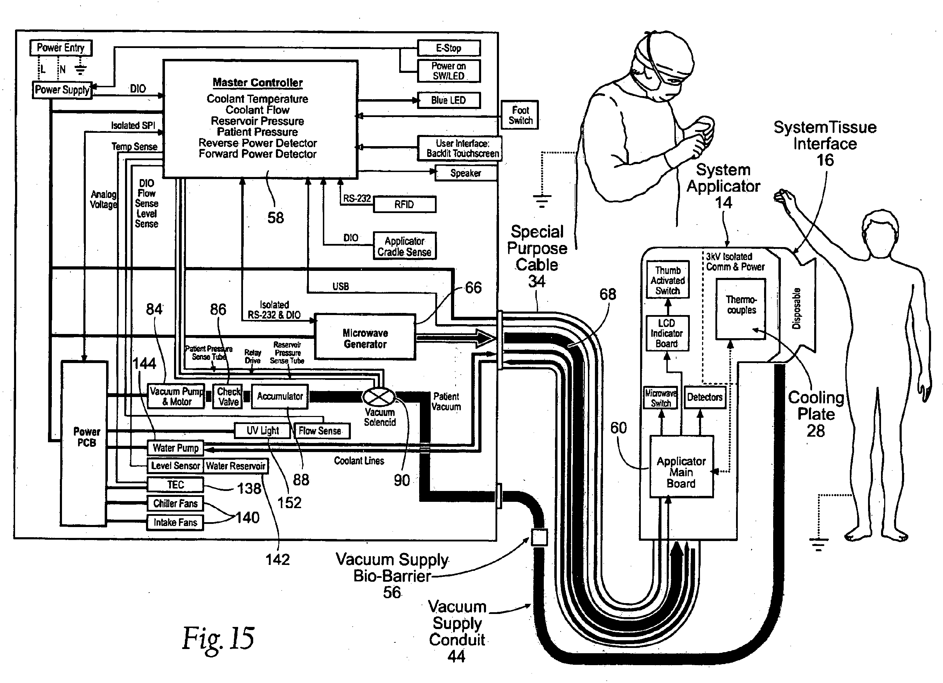

In the illustrative embodiment, the system console 12 may be a durable item capable of repeated reuse. As Figs. 1 to 4 show, the system console 12 comprises a cabinet or housing that is compact and capable of being wheeled for transport and positioning alongside an individual to be treated. Components housed within the console support specified treatment functions. An AC power cable 18 couples components within the system console 12 to a standard AC power outlet (see Fig. 4). A power supply within the system console 12 (see Fig. 15) converts the power to 12V DC power for distribution to the components housed within the system console 12.

In the illustrated embodiment, the specified system functions include an energy generation function; a tissue acquisition function; a lesion creation function; and a lesion control function.

B. The System Applicator

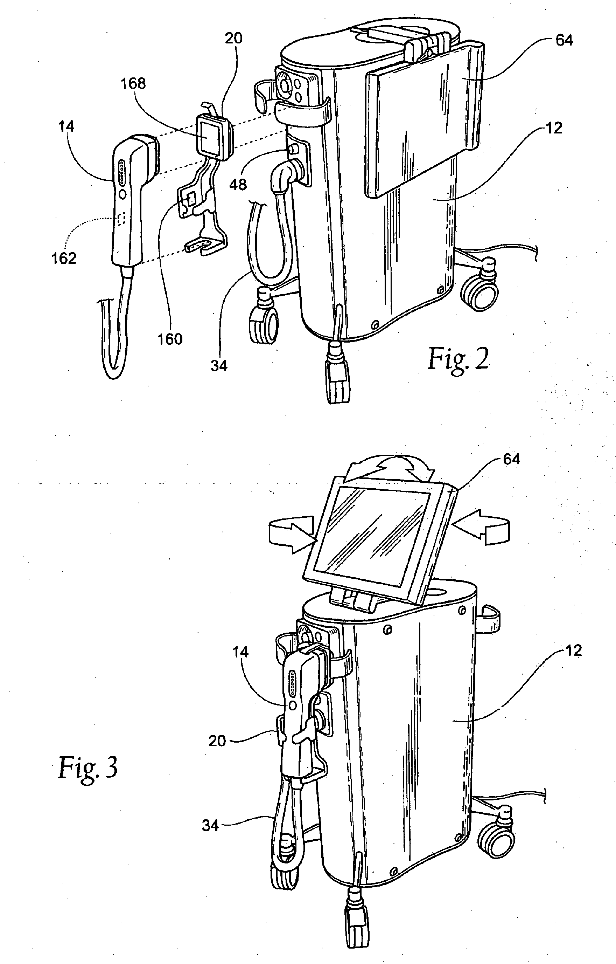

The system applicator 14 also may be a durable item capable of repeated re-use. The system applicator 14 may be sized and configured to be, during use, conveniently handled and manipulated in a hand of a caregiver (see Fig. 1). As Figs. 2 and 3 show, the system applicator 14 may be may be conveniently rested in a holster 20 on the system console 12.

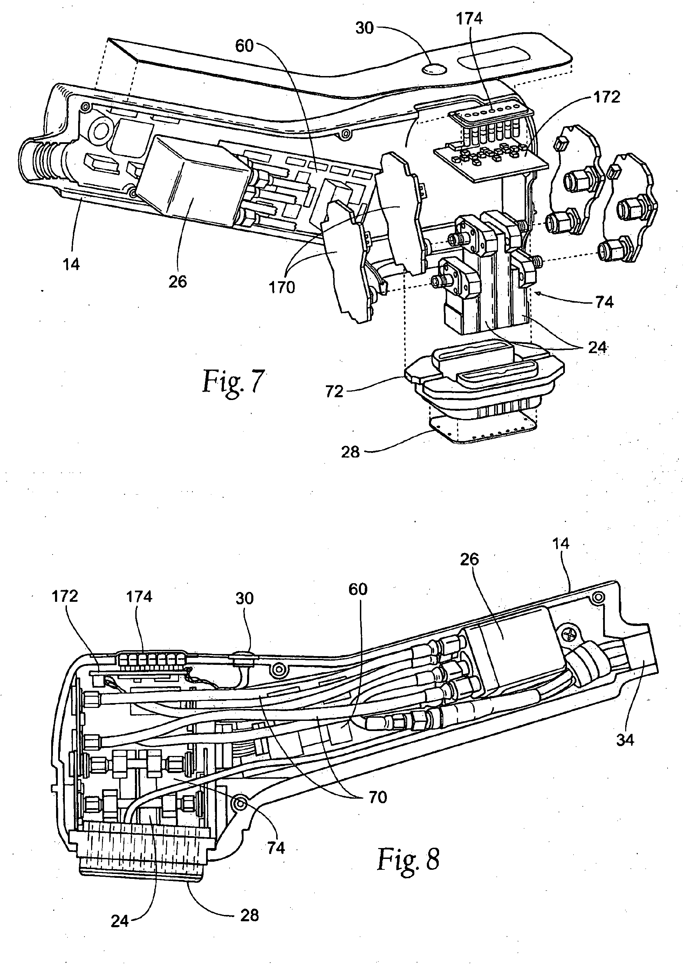

As shown in Figs. 1 and 5 to 8, the system applicator 14 comprises a pistol-grip housing made, e.g., of molded plastic material. Carried within the housing is a waveguide antenna array 22 (see Figs. 7 to 11). In the illustrated embodiment, the waveguide antenna array 22 comprises four waveguide antennas 24. It should be appreciated that the number of antennas 24 can vary according to the treatment objectives.

In use, the waveguide antenna array 22 radiates energy provided by the energy generation function.

Components in the applicator 14 also act in concert with components housed within the system console 12 to carry out the lesion generation and lesion control functions. More particularly, and will be described in greater detail later, the lesion generation function controlled within the console 12 operates a microwave switch 26 in the applicator 14 (see Fig. 7) to synchronize the radiation of energy by the antennas 24 in the applicator 14 to form desired patterns of lesions in the targeted tissue region (as Fig. 23B shows). Further, and as will also be described in more detail later, the lesion control function controlled within the console 12 provides a coolant that is circulated to a cooling plate 28 in the applicator 14 (see Fig. 7) that is in thermal conductive contact with the targeted tissue region. The temperature conditions of the cooling plate 28 control expansion of the lesion in the targeted tissue region.

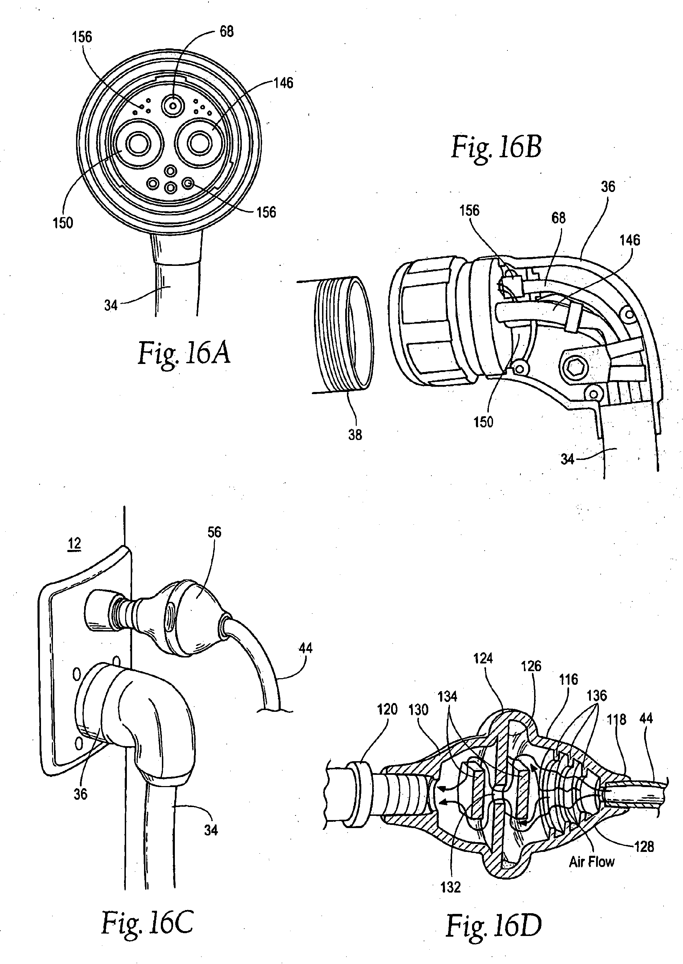

A "trigger" switch 30 on the system applicator 14 (see Fig. 7), which may, for example, be thumb actuated, gives the caregiver direct control over initiation and termination of treatment, subject to the overrides and global control of the master controller of the system console 12. Alternatively, or in combination, a foot pedal control switch 32 can be provided for the same purpose (see Fig. 1). A special purpose cable assembly 34 (see Figs. 1 and 16A/B) couples the components housed in the system applicator 14 to the components housed within the system console 12. The special purpose cable assembly 34 includes a custom designed multi-function plug 36 that couples to a dedicated connection 38 site on the system console 12.

C. The Applicator-Tissue Interface

The applicator-tissue interface 16 may be a single use, disposable item. More particularly, as shown in Figs. 6; 12A/B; and 13, the interface 16 may be sized and configured to be temporarily coupled to the system applicator 14 during use (e.g., by a latching mechanism 40), and then detached after use for disposal, as shown in Fig. 6. In this arrangement, the applicator-tissue interface 16 can, after an incidence of use, be detached from the system applicator 14, discarded, and replaced by another unused applicator-tissue interface 16 prior to a next incidence of use.

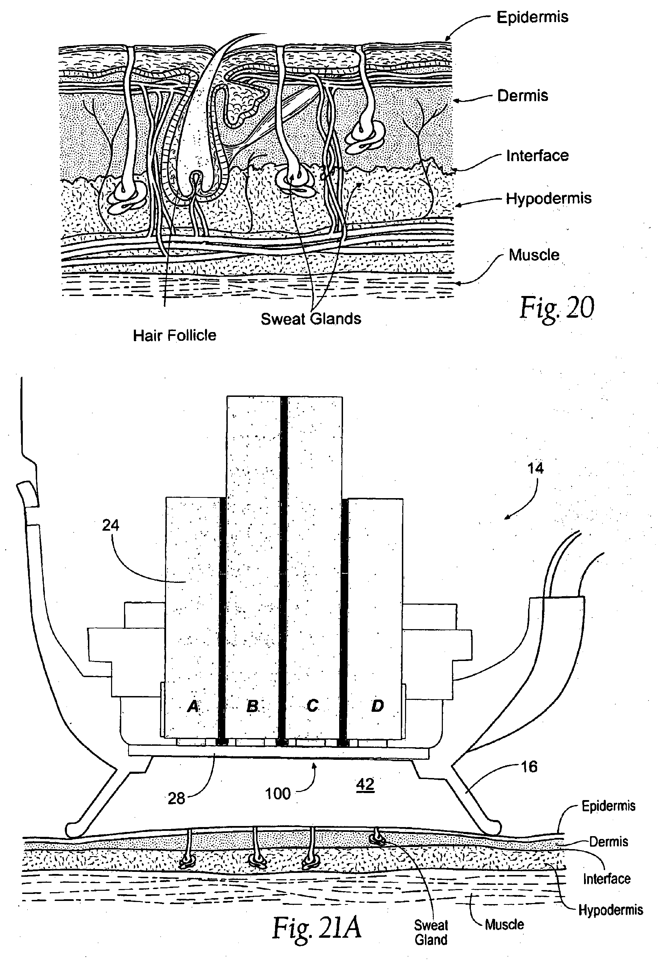

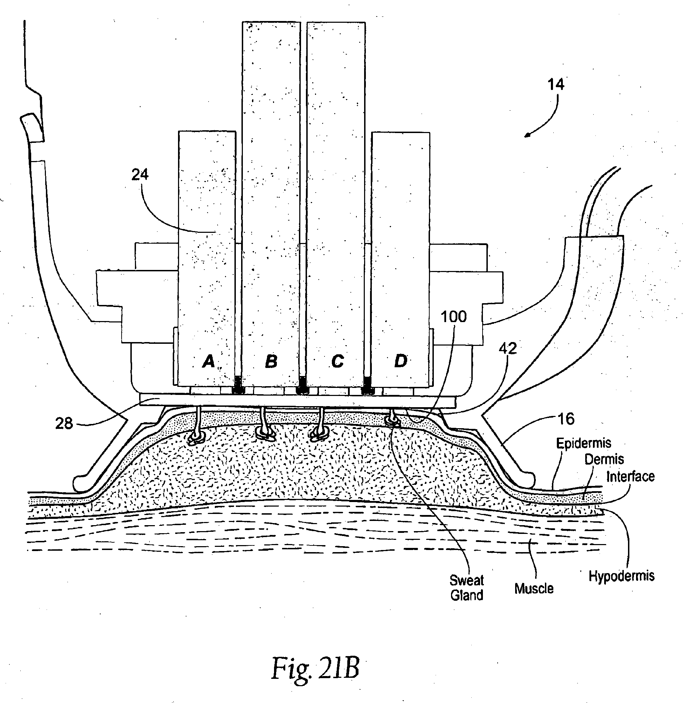

In use (e.g., see Figs. 21A and 21B), the applicator-tissue interface 16 of the present invention contacts the targeted tissue region and passes the energy radiated by the waveguide antenna array 22 to tissue. Components in the applicator-tissue interface 16 also act in concert with components housed within the system console 12 to carry out the tissue acquisition function. For this purpose, the applicator-tissue interface 16 includes a tissue acquisition chamber 42, into which tissue is drawn to elevate the dermis and hypodermis and localize and stabilize the targeted tissue region in thermal conductive contact with the cooling plate 28 as energy is applied from the waveguide antenna array 22. In the illustrated embodiment of the present invention the tissue acquisition function includes the application of a vacuum to the tissue acquisition chamber acquisition chamber 42. For this purpose, a vacuum supply conduit 44 couples the components housed in the applicator-tissue interface 16 to components housed within the system console 12. The vacuum supply conduit 44 plugs into a dedicated connection site 48 on the system console 12.

The application of the vacuum by the applicator-tissue interface 16, as controlled by the tissue acquisition function, provides uniformity and consistency in acquiring tissue for treatment. It reduces variability of treatment that may arise, e.g., due to differences in manipulation of the applicator by a given caregiver and/or difference among tissue topologies to be treated.

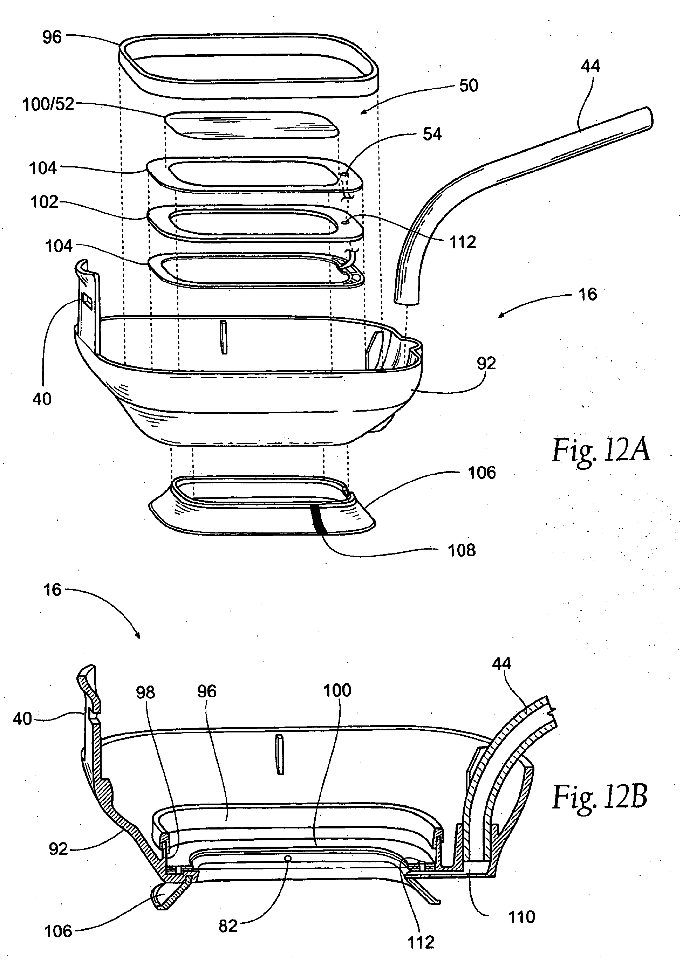

The applicator-tissue interface 16 also includes a multi-functional bio-barrier 50 (see Fig. 12A). As will be described in greater detail later, the multi-functional bio-barrier 50 isolates the operational components in the applicator 14 and the console 12 from contact with and contamination by physiologic liquids (e.g., blood and sweat) that may be present in the targeted tissue region. The multi-functional bio-barrier 50 substantially isolates the durable electrical and mechanical components of the system 10 (e.g., the applicator 14 and console 12), from the physiologic conditions of the tissue regions being treated, and vice versa.

II. The Functions of the System

As will be described, a master controller 58 housed on-board the system console 12 (see Fig. 15) monitors, controls, and coordinates the overall execution of the specified energy generation function, the tissue acquisition function, the lesion creation function, and the lesion control function by the system 10. The on-board master controller 58 serves to globally set and control output power, as well as the sequence of the application application of the waveform to the system applicator and waveguide antennas 24 within the applicator 14. The on-board master controller 58 also monitors operational conditions and initiates alarms when predetermined error or out of bound conditions occur.

An applicator control board 60 housed within the applicator 14 (see. e.g., Figs. 7 and 15) communicates with the master controller 58 and is controlled by the master controller 58 to support the energy generation function, the lesion creation function, and the lesion control function conducted by the applicator 14.

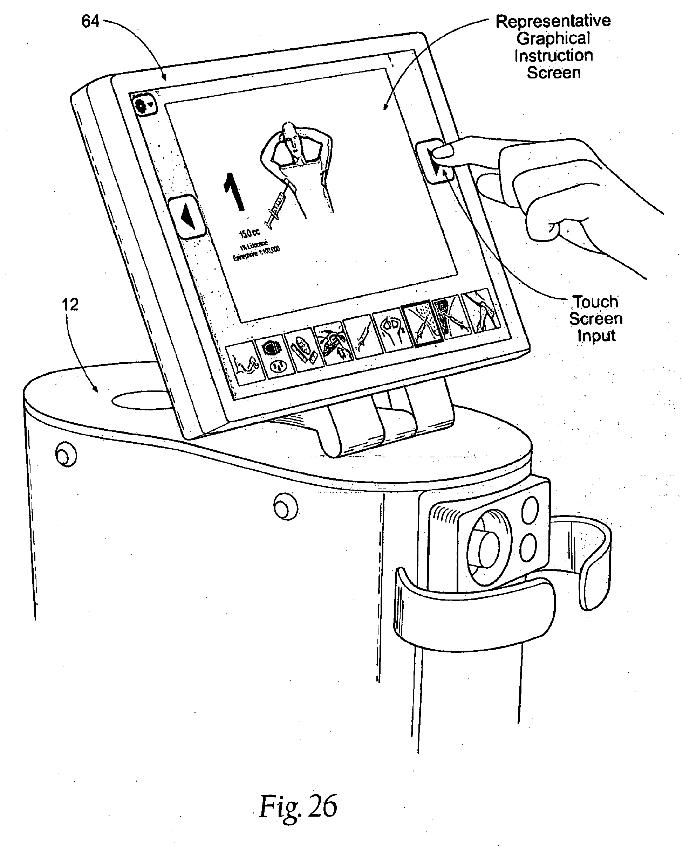

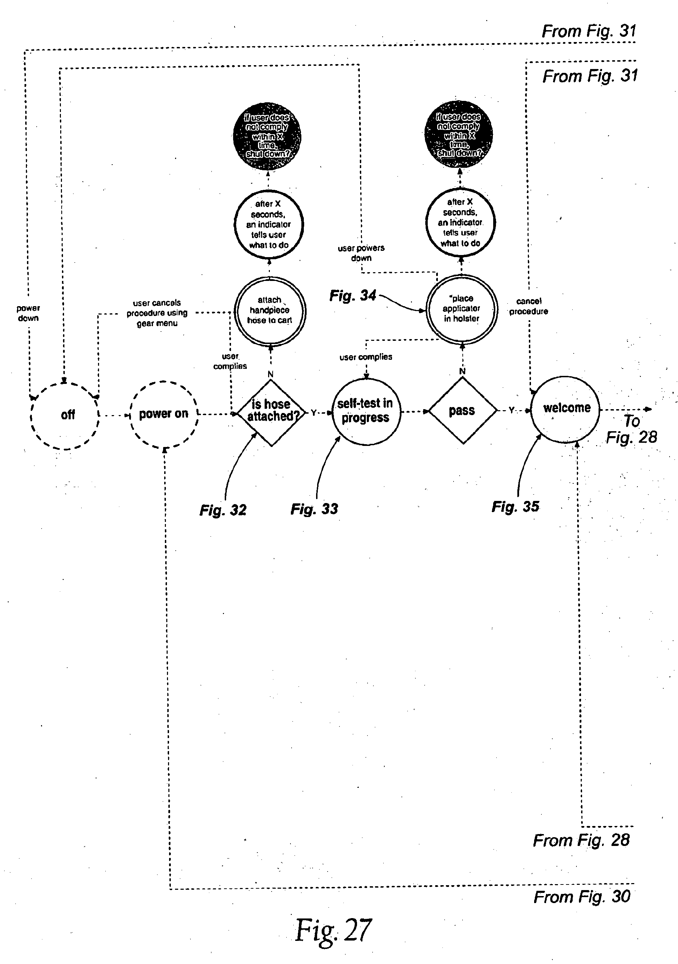

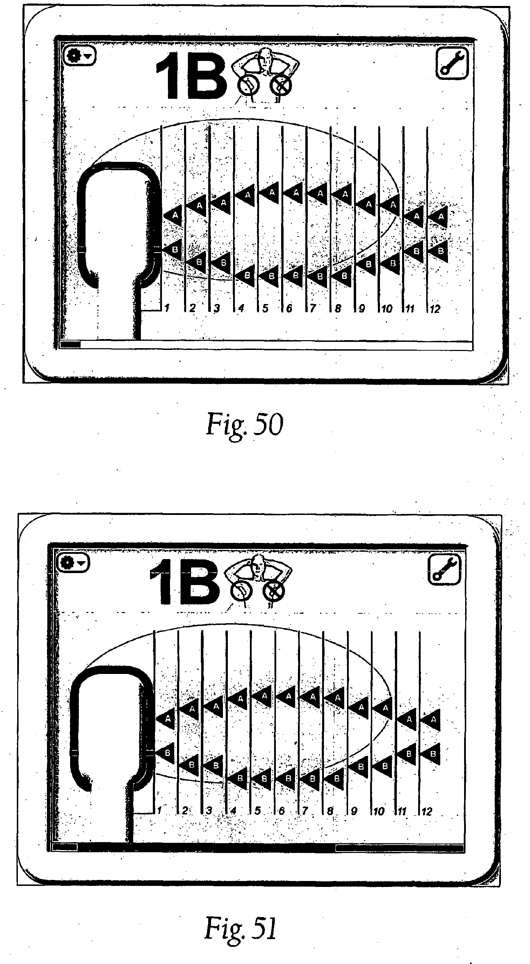

The master controller 58 may also implement a graphical user interface 62 (see, e.g., Fig. 26). The graphical user interface 62 may be generated on a display screen 64 that articulates on the system console 12, as Figs. 1 and 3 show). The graphical user interface 62 conveys status and operational information to the caregiver and allows the caregiver to provide control inputs. The graphical user interface 62 on the display screen 64 communicates the control and alarm conditions to the caregiver and allows for touch-screen interaction and input from the caregiver. Further details of a representative graphical user interface 62 will be described later (and, in particular, are shown in Figs. 27 to 59).

The energy generation function; the tissue acquisition function; the lesion creation function; and the lesion control function, as well as the principal cooperating components on the console 12, applicator 14, and applicator-tissue interface 16 that execute these functions will now be individually discussed in greater detail.

A. The Energy Generation Function

Components carried on-board the system console 12 (see Fig. 15) generate an energy waveform selected to achieve the desired therapeutic objective in the targeted tissue region. These components include a microwave generator 66 (Broadband Wireless, Model Number BW-5800-125-HS). The master controller 58 on-board the system console 12 includes preprogrammed rules or logic that set and/or vary the output power of the microwave generator 66 according to the therapeutic objectives of a given system.

Given the therapeutic objectives of treating hyperhidrosis, the microwave generator 66, under the control of the master controller 58, may generate at the time of treatment a microwave signal that lays in the ISM band of 5.775 to 5.825 GHz, with a frequency centered at approximately 5.8 GHz. Of course, other waveforms or variations in this waveform can be selected for generation by the waveform generation function. A microwave cable 68 in the special purpose cable assembly 34 couples the microwave signal to the system applicator 14.

The master controller 58 may set the power output for the microwave signal at between approximately 40 Watts and approximately 100 Watts, where the power output is measured into a 50 ohm load. As another example, the master controller 58 may set a power output at approximately 55 Watts measured into a 50 ohm load. The power output may be matched to the impedance of the system applicator 14, the special purpose cable assembly 34, and the applicator-tissue interface 16 to provide appropriate power out of the system applicator 14 at the frequency of interest.

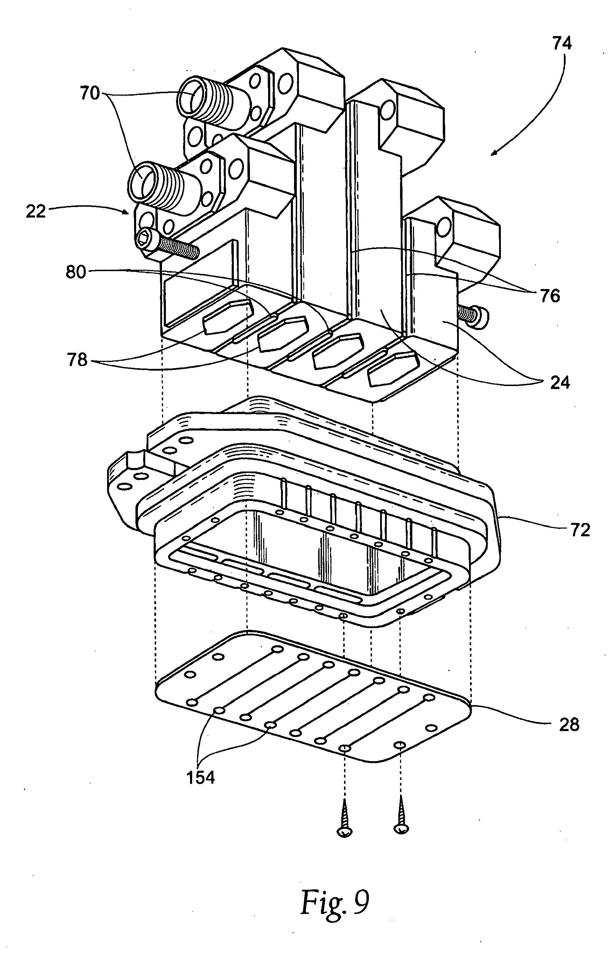

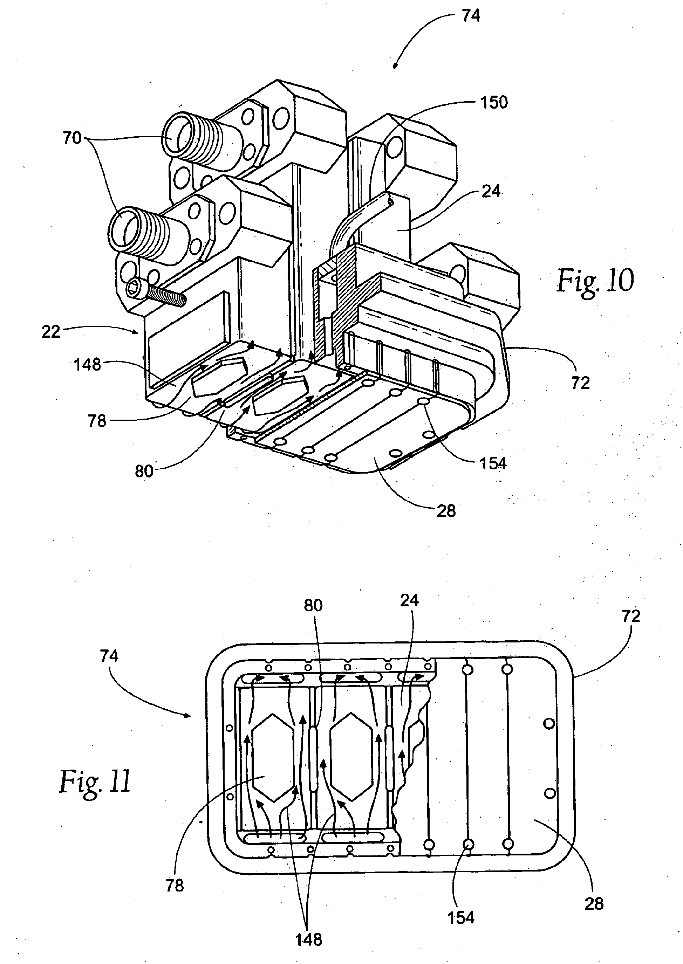

The system applicator 14 carries the waveguide antenna array 22 (see Figs. 9 and 10). The applicator also carries a microwave switch 26 (see Figs. 7 and 8) coupled to the microwave cable 68 of the special purpose cable assembly 34. Feed connectors 70 from the switch 26 couple couple to the four waveguide antennas 24 of the waveguide antenna array 22 (see Fig. 8).

The master controller 58 on-board the system console 12 includes preprogrammed rules or logic to distribute the microwave signal in a predetermined pattern to the waveguide antennas 24. Preprogrammed rules or logic on the applicator main board 60 convert the control signal pattern to switching signals, which are communicated to the microwave switch 26 in the applicator 14. In response, the antennas 24 radiate the microwave signal through the applicator-tissue interface 16 in a predetermined pattern (controlled by the lesion generation function) to form prescribed lesion patterns in the targeted tissue region (as shown, e.g., in Fig. 23B).

The assembly of the waveguide antenna array 22 can vary. In the representative illustrated embodiment (see, in particular, Figs. 9 and 10), the array of waveguide antennas 24 is supported within the system applicator 14 by an antenna cradle 72 and waveguide assembly 74. A cooling plate 28 supported on the antenna cradle 72 faces the applicator-tissue interface 16. As will be described in greater detail later, the cooling plate 28 is one component of a cooling system controlled by the master controller 58 that may serve to control lesion formation, by, for example, preventing lesions from expanding toward the surface of the skin as they are formed by the applied microwave energy.

In the illustrated embodiment (see Fig. 9), the waveguide assembly includes spacers 76 (which may be, for example, a metal material such as copper or aluminum shims) positioned between waveguide antennas 24. The thickness of the spacers 76 is selected to manipulate the shape of the power distribution pattern applied when, for example, adjacent waveguide, antennas 24 are commanded to radiate power (which is called a phase drive mode, as will be described in greater detail later). As shown in Fig. 9, the heights of waveguide antennas 24 in the waveguide antenna array 22 are staggered to facilitate access to the feed connectors 70. Each waveguide antenna 24 may be manufactured by coating a dielectric center region with a metal material, e.g., copper or nickel. The thickness of the metal material may, at a minimum, correspond to the skin depth of the applied microwave energy at the frequency of interest, e.g., 5.8 GHz. Typically, the thickness is significantly greater, e.g., 0.00635 mm (.00025 inches) or more.

In the illustrated embodiment (see Fig. 9), each waveguide antenna 24 may include at least one scattering element 78, which projects from its lower, tissue facing surface, which can also be called the antenna aperture. The scattering elements 78 are sized and configured to optimize the size and shape the lesions. In the illustrated embodiment, each scattering element 78 projects toward tissue generally from the center of the respective waveguide antenna aperture. Still, in an alternative embodiment, the scattering element 78 need not be centered on the waveguide antenna aperture. The scattering element 78 may project about 1 mm from the aperture.

In the illustrated embodiment (see Fig. 9), intermediate scattering elements 80 may be positioned between the waveguide antennas 24. The intermediate scattering elements 80 may be sized and configured to optimize the size and shape of lesions developed in the skin between waveguide antennas 24, for example, by improving the Specific Absorption Rate (SAR) pattern in tissue. By altering the material, size, and configuration of the intermediate scattering elements 80, lesions created in tissue by the waveguide antennas 24 can be made made larger and more spread out, or (conversely) narrower, depending upon the therapeutic objectives. For example, increasing the dielectric constant of an intermediate scattering element 80 may reduce the size of a lesion created in skin between waveguide antennas 24, and vice versa.

The intermediate scattering elements 80 may be manufactured from, for example, alumina or from a material that is approximately 96% alumina. Alternatively, the intermediate scattering elements 80 may be manufactured from, for example, silicone or injected molded silicone. The intermediate scattering elements 80 may be manufactured from a material having approximately the same dielectric constant as the scattering elements 78, e.g., a dielectric constant of approximately 10, and more preferred, a dielectric constant of approximately 3.

The intermediate scattering elements 80 may be sized such that they have a width which is not more than slightly wider than the separation distance between apertures of the waveguide antennas 24, so that they do not substantially interfere with the radiated energy. The intermediate scattering elements 80 may be sized and configured to modify and/or spread out the radiated microwave field.

In a representative embodiment, the intermediate scattering elements 80 may have an optimal length which is shorter than the length of scattering elements 78, e.g., approximately 7 mm in length, or more preferred 6.3 mm in length.

1. The Tissue Acquisition Function of the present invention

Components carried on-board the system console 12 (see Fig. 15) generate negative pressure that is communicated to the applicator-tissue interface 16 by the vacuum supply conduit 44. As will be described in greater detail later (and as generally illustrated in Fig. 12B), the applicator-tissue interface 16 includes a formed tissue acquisition chamber 42 with ports 82 through which negative pressure is directed by the vacuum supply conduit 44 to draw tissue into the acquisition chamber 42, as Fig, 21B shows). The negative pressure applied to tissue in the acquisition chamber 42 localizes and stabilizes the tissue while microwave energy is applied.

The tissue acquisition function can be accomplished in concert with the tissue acquisition chamber 42 in various ways. In the illustrated embodiment (see Fig. 15), the tissue acquisition function includes a motor-driven vacuum pump 84 coupled via a one-way check valve 86 and accumulator (reservoir) 88 to a solenoid vacuum valve 90. The check valve 86 between the vacuum pump 84 and the accumulator 88 allows the vacuum pump 84 to be shut off when no additional vacuum is required. The accumulator (reservoir) 88 may accommodate, e.g., at least 491,6 cm$^3$ (30 cubic inches) in volume to provide a large capacity of vacuum.

The vacuum pump 84 may comprise, e.g., a scroll vacuum pump with a brushless DC motor (Air Squared Model No. V11H12N2.5). The solenoid vacuum valve 90 may comprise. e.g., a solenoid valve, three way, normally closed, exhaust to atmosphere (Model LW53KK8DGBG12/DC, Peter Paul Electronics, Co.). The vacuum pump 84 maintains, e.g., a vacuum level of between minus 67,7 kPa (20 inches) to minus 74,5 kPa (22 inches) of Hg for proper tissue acquisition.

As Fig. 15 shows, the motor-driven vacuum pump 84 may receive power through the power supply and power printed circuit board (PCB) within the system console 12. The solenoid vacuum valve 90 may also be coupled to and controlled by the master controller 58 onboard the system console 12, so that its operation can be coordinated by coordinated by the master controller 58 with the generation and application of microwave energy, as well as other functions of the system 10.

The motor-driven vacuum pump 84 creates negative pressure. The solenoid vacuum valve 90 communicates with the vacuum supply conduit 44. When opened by the master controller 58, the solenoid vacuum valve 90 conveys negative pressure generated by the vacuum pump 84 to the tissue acquisition chamber 42 of the applicator-tissue interface 16. Closing the solenoid vacuum valve 90 interrupts the supply of negative pressure to the applicator-tissue interface 16.

Referring now to Figs. 12A and 12B, the applicator-tissue interface 16 may comprise a body 92 formed from a medical grade rigid or semi-rigid plastic material, e.g., polycarbonate. The body 92 may be formed, e.g., by molding, into a bowl shape. Latching assembly 40 can be integrally formed on the body 92 to couple to a mating attachment member 94 on the system applicator 14 (see, e.g., Fig. 5), to fasten the applicator-tissue interface 16 to the system applicator 14 at time of use and disconnect the interface from the system applicator 14 after use (as Figs. 5 and 6 illustrate).

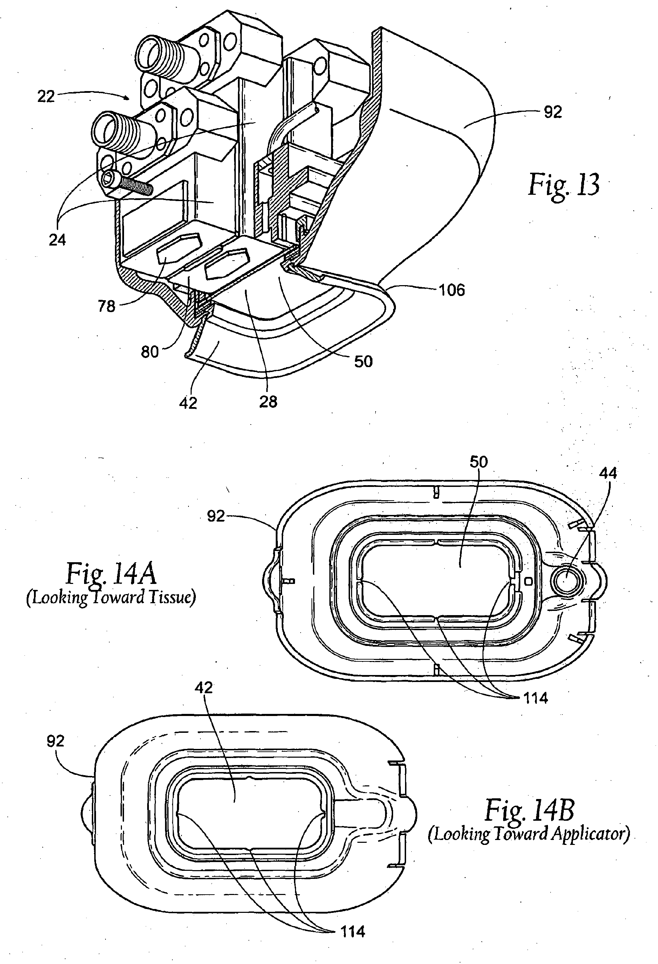

Within the bowl shaped body 92 (as best shown in Fig. 12B), a waveguide holder gasket 96 is seated on peripheral flange 98 formed in the bowl. The waveguide holder gasket 96 is sized and configured, when the interface body 92 is fastened to the system applicator 14 (see Fig. 13), to form a fluid-tight, pressure-tight seal against the periphery of the cooling plate 28 on the undersurface of the waveguide assembly.

Within the bowl shape body 92 (see Figs. 12A and 12B), spaced below and inward of the waveguide holder gasket 96, is a tissue interface surface 100. In the illustrated embodiment (as best shown in Fig. 12A), the tissue interface surface 100 comprises a frame 102 with upper and lower overlying adhesive panels 104. A first bio-barrier component 52 is mounted on the upper adhesive panel, and the lower adhesive panel adheres to an interface surface support in the bowl, which the waveguide holder gasket 96 peripherally surrounds.

In use, tissue being treated contacts the first bio-barrier component 52 in thermal contact with at least a portion of the cooling plate 28. The first bio-barrier component 52 forms a part of the multi-functional bio-barrier 50 of the applicator-tissue interface 16. The first bio-barrier component 52 forms comprises the actual tissue surface interface, which tissue acquired within the tissue acquisition chamber 42 contacts as energy is applied from the waveguide antenna array 22. The first bio-barrier comprises 52 a material that is selected on the basis of different, but overlapping physical criteria.

One selection criteria for the first bio-barrier component 52 is that the material is substantially impermeable to both air and liquids, such as blood and/or sweat, which may be present in the tissue acquisition chamber 42. As the tissue acquisition function applies vacuum to draw tissue within the tissue acquisition chamber 42 into contact with the first bio-barrier component 52, the first bio-barrier component 52 isolates the components in the applicator 14 from contact with and contamination by physiologic liquid in the targeted tissue region.

An overlapping selection criteria for the first bio-barrier component 52 is that the material, taking into account its thickness, possesses requisite low microwave conductivity, so that it efficiently passes the microwave energy radiated by the waveguide antenna array 22 to the targeted tissue region acquired within the tissue acquisition chamber 42, with minimal energy absorption. This characteristic can be expressed as a loss tangent tan $\delta$ of 0.1 or less, and more desirably approximately 0.0004.

The loss tangent tan $\delta$ is similar to conductivity $\sigma$, but also takes into account the dielectric constant of the material, as follows:

$$\tan\,\delta\,=\,\sigma/\omega\varepsilon$$

where $\omega$ is frequency, and
where $\varepsilon$ is permittivity

For example, at 5.8 Ghz, a range of conductivities $\sigma$ suitable for use as the first bio-barrier component, 52, corresponding to a tan $\delta$ equal to or less than 0.1, would be $\sigma = 0.0$ to $0..2$ siemens/meter.

Another overlapping selection criteria for the first bio-barrier component 52 is that the material, taking into account its thickness, possesses requisite high thermal conductivity, to efficiently allow thermal conduction to occur between the targeted tissue region acquiring within the tissue acquisition chamber 42 and the cooling plate 28. For example, the material selected should have a thermal conductivity of at least 0.1 watts per meter-Kelvin (0.1 W/mK), and desirably 0.1 to 0.6 W/mK, and most desirably 0.25 to 0.45 W/mK.

Another overlapping selection criteria for the first bio-barrier component 52 is that the material, taking into account its thickness, possesses requisite high heat transfer coefficient. The heat transfer coefficient can be expressed by the thermal conductivity of the material divided by the thickness of the material. For example, for a first bio-barrier component 52 with a thermal conductivity of 0.1 and a thickness of 0.127 mm (.0005 inches) the heat transfer coefficient would be about 7874 W/m2K.

Other overlapping selection criteria for the first bio-barrier component 52 is that the material is sufficiently flexible to conform to the surface of the cooling plate 28, while also being sufficiently strong to resist tearing as a result of vacuum pressure or contact with tissue.

In this respect, the first bio-barrier component 52 may be a nonporous membrane, e.g., polyethylene film, nylon, or other suitable materials. The first bio-barrier component 52 is desirably flexible and soft for compliant contact with skin. The first bio-barrier component 52 can comprise, e.g., polyethylene film available from Fisher Scientific, or (alternatively) Mylar film. The bio-barrier component 52 can be, e.g., about 0.127 mm (.0005 inch) in thickness.

The applicator-tissue interface body 92 also includes a skirt 106 (see Figs. 12A/B and 13) that depends downwardly with an increasing diameter from the body about the periphery of the applicator-tissue interface surface. The downward depending skirt 106 defines a generally funnel-shaped open interior area or chamber leading to the first bio-barrier component 52 of the applicator-tissue interface 16 (see Fig. 13). This chamber defines the tissue acquisition chamber 42 previously described. The skirt 106 may comprise a compliant medical grade plastic material (e.g., a thermal plastic elastomer (TPE) such as urethane; or silicone; or natural or synthetic rubber; or an elastomeric material) and may be sized and configure to rest comfortably against an external skin surface. When pressed with sufficient pressure to compress against a tissue surface (see Fig. 21A), the periphery of the skirt 106 forms a generally fluid-tight, pressure-tight seal about the tissue acquisition chamber 42.

The skirt 106 may include an alignment member 108 (see Fig. 5) positioned on each side of the skirt 106 to provide a positioning point of reference to the caregiver during manipulation of the interface, as will be described in greater detail later. The funnel-shaped contour of the skirt 106 may provide a skirt angle that gives the caregiver a direct view of the alignment members 108, while the caregiver manipulates the applicator-tissue interface 16 attached to the system applicator 14.

As Fig. 12A shows, the vacuum supply conduit 44 communicating with the tissue acquisition function of the system console 12 (see also Fig. 15) is coupled to a port formed on the body of the applicator-tissue interface 16. The port communicates with a vacuum channel 110 formed in the body (see Figs. 12A and 12B) that communicates with the tissue acquisition chamber 42 adjacent the applicator-tissue interface surface. The vacuum channel 110 may circumferentially encircle the tissue acquisition channel at or near the applicator-tissue interface surface. The vacuum channel 110 may include spaced-apart apertures or ports 82 formed along the vacuum channel (see Fig. 12B) (e.g. four ports, one adjacent each side the applicator-tissue interface surface), to convey negative pressure uniformly into the tissue acquisition chamber 42 adjacent the applicator-tissue interface surface. The ports 82 suction skin into the chamber and position the skin against the first bio-barrier component 52 in thermal conductive contact with the cooling plate 28 (as Fig. 21B shows).

The frame 102 and panels 104 of the tissue interface surface 100/52 may include formed apertures 112 (see Fig. 12A) that register when assembled to form a vacuum balance path that communicates with the tissue acquisition chamber 42. Negative pressure applied in the chamber 42 is conveyed through the vacuum balance path 112 to the opposite side of the interface surface 100/52 to equalize pressure on both sides of the interface surface 100/52. surface 100/52.

A second bio-barrier component 54 of the multifunctional bio-barrier 50 of the applicator-tissue interface 16 desirably occupies the vacuum balance path 112. The second bio-barrier component 54 in the vacuum balance path 112 (which can also be called the "vacuum balance bio-barrier component") comprises a material that is substantially impervious to liquid, but not to air. The vacuum balance bio-barrier component 54 prevents physiologic liquids such as blood and/or sweat that may be present in the tissue acquisition chamber 42 from being transported through the vacuum balance path 112 into the interior of the applicator 14. Candidate materials for the second bio-barrier component 54 may include pores sufficient to pass air (e.g., 0.45 μm) to substantially equalize the vacuum pressure on the system applicator side and the interface side of the surface, without passing biological liquids from the acquisition chamber 42 into the system applicator 14. The second bio-barrier component 54 may comprise, e.g., a hydrophobic membrane made from PTFE (Teflon) material. The second bio-barrier component 54 can be, e.g., about 0.127 mm (.005 inch) in thickness.

The spaced-apart apertures or ports 82 formed along the vacuum channel may include interior patterns 114 along its interior that can impress a "hickey pattern" on the skin drawn into the chamber 42 (see Figs. 14A and 14B). The existence of "hickey patterns" with the lesions can help guide the caregiver in successive placements of the system applicator 14 to accurately place a succession of lesions in the targeted tissue region. Inconsistencies in the "hickey patterns" may also alert the caregiver to gaps or inaccurately placed lesions in the lesion pattern, to indicate a need to return and fill in gaps and missed spots in the pattern. The interior patterns 114 define The interior patterns 114 define notches that break surface contact and may help to prevent skin from blocking the vacuum apertures or ports.

In a representative embodiment, the tissue acquisition chamber 42 is dimensioned approximately 39 mm (1.54 inches) by approximately 17,8 mm (0.7 inches) having a depth (without the skirt 106) of approximately (0.177 inch) 4.5 mm. With the skirt 106, the depth of tissue acquisition chamber 42 can be between approximately 6.5 mm to 11 mm, depending upon the extent to which the compliant skirt 106 is compressed against the skin by the application of vacuum. According to an embodiment of the invention the four corners of the tissue acquisition chamber 42 may have a radius of 4,76 mm (1875 inches).

In this arrangement, the waveguide antenna array 22 on the opposite side of the tissue interface surface 100/52 include four antennas 24 and possesses dimensions of approximately 34 mm (1.34 inches) by approximately 15,95 mm (0.628 inches). The dimensions of the waveguide antenna array 22 and the tissue acquisition chamber 42 are desirably optimized to minimize stray fields forming at the edges of waveguide antenna array 22, as well as optimizing the effective cooling area of the tissue interface surface. The tissue acquisition chamber 42 is desirably optimized to facilitate tissue acquisition without adversely impacting cooling or energy transmission.

The vacuum supply conduit 44 may collect liquids (e.g., sweat or blood) that escape during the treatment process. For this reason, a third bio-barrier component 56 of the multi-functional bio-barrier 50 of the applicator-tissue interface 16 is placed upstream of the applicator-tissue interface 16 in-line in the vacuum supply conduit 44 (see Fig. 15, as is also generally shown in Fig. 1). The third bio-barrier component 56 is selected to be substantially impervious to liquid, but not to air. The not to air. The third bio-barrier component 56 can comprise, e.g., a hydrophobic filter (e.g., a Millex FH filter made of 0.45 μm hydrophobic PTFE available from Millipore) to keep liquids out of the system console 12. The hydrophobic filter can be further characterized, e.g., by accommodating an airflow of approximately 0.38 m³/min (13.4 cubic feet per minute) at approximately 69 kPa (10 pounds per square inch).

The third bio-barrier component 56 can, alternatively, comprise an in-line vacuum trap, as shown in Figs. 16C and 16D. The vacuum trap may include a formed housing 116 defining a vacuum inlet port 118 (with which the vacuum supply conduit 44 communicates) and a vacuum outlet port 120 (which plugs into the connection site 48 on the console 12). The housing 116 defines an interior chamber 122, which the vacuum flow between the inlet and outlet ports 118 and 120 must traverse from the applicator- tissue interface 16 to the system console 12. A central ridge 124 on the exterior of the housing 116 may provide a gripping surface for the caregiver to hold and manipulate the vacuum trap, e.g., while plugging the vacuum supply connector 118 into and out of the mating console vacuum supply receptacle 48.

The chamber 122 is compartmentalized by an interior wall 126 into an inlet side 128, communicating with the inlet port 118, and an outlet side 130, communicating with the outlet port 120. One or more apertures 132 in the interior wall 130 define path(s) of flow communication between the inlet and outlet sides lw28 and 130 of the chamber 122.

Baffle plates 134 interfere with vacuum flow through the aperture (s) 132 through the interior wall 16 between the inlet side 128 and outlet side 130 of the chamber 122. The vacuum flow must veer around the baffle plates 134 to transit through the chamber 122. An array of annular annular annular baffles 136 is further circumferentially placed around the inlet side 128 of the chamber 122. The baffle plates 134 and annular baffles 136 form an array of tortuous paths, through which vacuum flow transiting the chamber must navigate. Air in the vacuum flow will readily change direction to navigate the tortuous paths. Physiologic liquid carried by the vacuum flow will not, and will instead be captured by gravity in the nooks and crannies of the tortuous paths through the chamber 122. -The vacuum trap thereby prevents physiologic liquid from passing out of the outlet port 120 into the console 12.

2. The Lesion Creation Function

As will be described in greater detail later, the microwave signal applied through the waveguide antennas 24 to tissue acquired within the tissue acquisition chamber 42 creates lesions in the targeted tissue region, as generally shown in Fig. 23B. In the treatment of hyperhidrosis, the lesions may be formed in the lower dermis and/or dermis/hypodermis of the skin. Components carried on-board the system console 12 control the microwave switch 26 carried on-board the applicator 14 to sequence the application of the microwave signal by the waveguide antennas 24 in prescribed manners to form the lesions in selected patterns, as Fig. 23B illustrates. The patterns are selected to be conducive to achieving the therapeutic objectives of the system 10.

(iv) The Lesion Control function

Components carried on-board the system console 12 (see Fig. 15) generate and circulate cooling fluid through coolant paths between the waveguide antenna array 22 and tissue cooling plate 28 carried in the system applicator 14, as is generally shown in Figs. 10 and 11). The tissue cooling plate 28 protects skin engaged with the applicator-tissue interface 16 (see Fig. 21B) from thermal damage by preventing lesions formed in the dermis/hyperdermis from expanding toward the epidermis.

The cooling fluid may comprise, e.g., water, deionized water, or other suitable fluid.

The lesion control function can be accomplished in various ways. In the illustrated embodiment (see Fig. 15), the lesion control function includes a Peltier-effect thermoelectric cooler (TEC) 138. As Fig. 15 shows, the TEC 138 receives power through the power supply and power printed circuit board carried on-board the system console 12, as do intake fans and chiller fans 140 that circulate air within the console 12 for conveying from the console 12 heat generated by the components, including the TEC 138. The TEC 138 chills coolant in a reservoir 142 on-board the console 12. A water pump 144 (also drawing power via the power printed circuit) conveys chilled coolant from the reservoir 142. The chilled coolant is circulated by a coolant supply line 146 through coolant paths 148 (see Figs. 10 and 11) in the waveguide antenna cradle 72 and between the waveguide antenna array 22 and tissue cooling plate 28 carried in the system applicator 14. Coolant is returned by a coolant return line 150 to the reservoir 142. The coolant supply and return lines 146 and 150 extend through the special purpose cable assembly 34 (see Figs. 16A and 16B) to the applicator 14. A cooling fluid germicidal lamp 152 (e.g., 253.7 nm) may be provided to prevent growth of microorganisms contaminants in the coolant that could clog the fluid lines and reduce coolant flow in the applicator 12. The cooling fluid germicidal lamp 152 may be activated periodically, e.g., for a period of time (e.g., 10 minutes) following each power-on cycle or each coolant refill.

As Fig. 15 shows, the TEC 138 may be coupled to and controlled by the master controller 58, so that its operation (like that of the components of the tissue acquisition function) can be coordinated by the master controller 58 with the generation and application of microwave energy.

In the illustrated embodiment, the cooling plate 28 rests against the terminal surfaces of the scattering elements 78 (see Figs. 10 and 11), but, in an alternate embodiment, the terminal surfaces of the scattering elements can be spaced out of contact with the cooling plate 28. Cooling paths 148 for the waveguide antenna array 22 are formed in the spaces between the cooling plate 28 and each waveguide antenna 24, into which the scattering elements 78 project. Coolant is circulated through these paths 148, cooling each waveguide antenna 24 individually and the cooling plate 28 in general.

The flow rate of coolant through these paths 148 can be, e.g., approximately 425 milliliters per minute +/- 45 milliliters per minute. Desirably, the paths 148 are sized and configured so that the flow rate of coolant along each waveguide antenna 24 is substantially the same. The temperature of the coolant can be, e.g., between approximately 8 degrees centigrade and approximately 22 degrees centigrade, and preferably approximately 15 degrees centigrade.

The scattering elements 78 may extend into at least a portion of coolant paths 148. It is desirable that the cooling paths 148 be smoothed or rounded or shaped in the manner shown in Figs. 9, 10, and 11 to reduce the generation and/or build up of air bubbles in the paths 148. The scattering elements 78, for example, may be formed in the shape of ovals or racetracks or oblong hexagons with "faceted" or tapering ends (see Figs. 9 to 11). Hydrophilic coatings may be used on some or all of the cooling paths 148 to, e.g., reduce the formation of bubbles.

The intermediate scattering elements 80 associated with the waveguide antenna array 22 may also be located in the coolant paths 148 between the antenna apertures. In this arrangement, the intermediate scattering elements 80 may be positioned such that they facilitate equalized cooling across the cooling plate 28, keeping in mind, however, that their principal function is to influence lesion size and shape in, for example, the phase drive mode. The intermediate scattering elements 80 may be sized such that they have a width which is not more than slightly wider than the separation distance between apertures of the waveguide antennas 24, so that they do not substantially interfere with the radiated energy. The intermediate scattering elements 80, which extend into coolant paths 148, may likewise be smoothed or rounded or shaped in the manner shown in Figs. 9, 10, and 11 to prevent the generation and/or buildup of bubbles in the paths. The intermediate scattering elements 80, for example, may be formed in the shape of ovals or racetracks or oblong hexagons with "faceted" or tapering ends (see Fig. 9 to 11), provided that they are sized and configured to modify and/or spread out a microwave field as it travels through the coolant path 148. In this arrangement, the intermediate scattering elements 80 (and the scattering elements 78) are desirably made of materials which will not rust or degrade upon exposure to the coolant.

Likewise, the cooling plate 28 may be laser cut (with a thickness, e.g., of about 0,51 mm (.020 inch) with curved corners.

Thermocouples 154 may be placed on the surface of the cooling plate 28 opposite to the cooling path 148 (see Figs. 10 and 11), generally aligned with and between the apertures of the waveguide antennas 24. The thermocouples 154 can, if desired, be printed (sputtered) on the cooling plate 28. The thermocouples 154 can, e.g., comprise a . comprise a plurality of T-type thermocouples (e.g., seven) masked and sputtered with varying alloy compositions of copper arid nickel, e.g., constantan, such as, e.g., 60% copper/40% nickel, and then re-masked and sputtered with copper such that the copper and the copper-nickel components form a junction that serves as a thermocouple. One or more thermocouples 154 may also be placed in the supply and return lines. The thermocouples 154 are coupled to the applicator main board 60, which communicates sensed temperature conditions through the special purpose cable assembly 34 to the master controller 58 on board the system console 12. The sensed temperature conditions are processed according to preprogrammed logic residing in the master controller 58, as part of the waveform generation function, and may be used to adjust power supplied to the waveguide antennas 24 based upon temperature conditions sensed along the cooling plate 28.

III. System Controllers

A. On Board the System Console

(The Master Controller)

The master controller 58 (see Fig. 15) resides on a control printed circuit board in the system console 12. The master controller 58 receives 12V power from the power supply. The master controller 58 communicates with components carried by the system console 12 as well as components carried by the applicator.

The special purpose cable assembly 38 (see Fig. 1 and 16A/B) establishes a multi-purpose link between the master controller 58 and the applicator 14. Extending through the special purpose cable assembly (see Figs. 16A and 16B) are the microwave energy cable 68 for conveying the microwave signal from the generator controlled by the master controller 58 to the microwave switch 26 on the applicator 14; the coolant supply and return conduits 146 and 150 for conveying coolant to and from the coolant reservoir 142 for circulation through the coolant paths 148 of the waveguide antenna array 22 and cooling plate 28 in the applicator; and a connector 156 establishing communication links between the master controller 58 and the applicator main control board 60 according to a prescribed communication protocol (e.g., the CMX-RTX™ RTOS (embedded real-time operating system) Product Line, available from CMX Systems, San Jose, California). The bundling of multiple electrical and fluid conduits through a single special purpose cable assembly 34 serves to streamline the form and function of the system 10 and simplify set up of the system 10, while also efficiently supporting the diverse functions of the system 10 itself, in terms of electrical waveform generation, coolant circulation, and providing communication links. The multi-special purpose cable assembly 34 facilitates lengthening the cable (e.g., upwards to 24 m (eight feet)) for better reach and ease of manipulation remote from the system console 12.

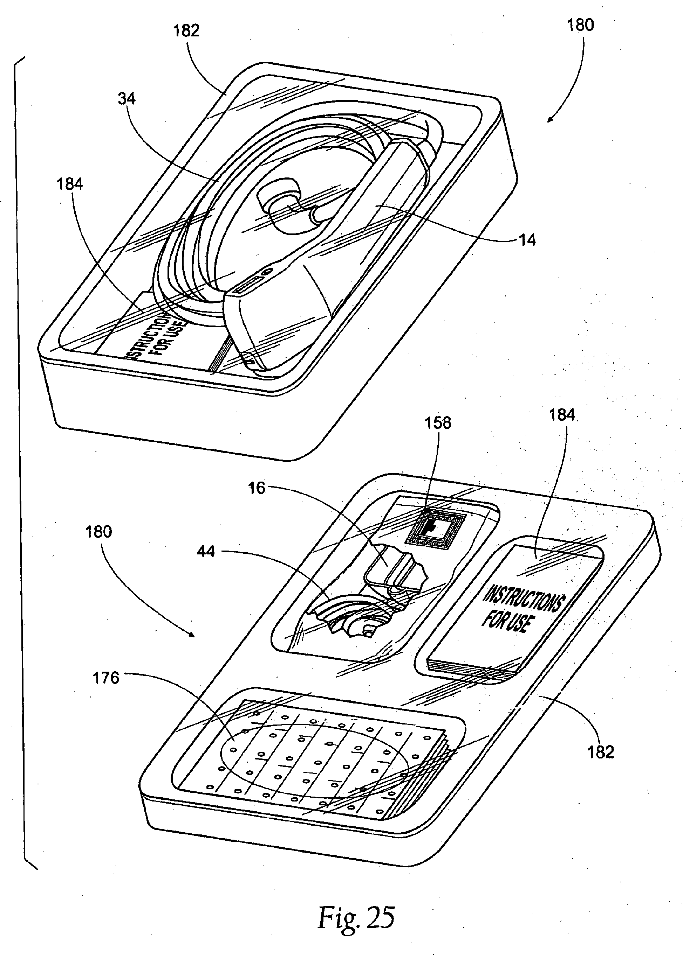

As shown in Fig. 15, communications between the master controller 58 and components residing in or on the console 12 can include (i) the colored (e.g., blue) LED on the console 12 that indicates when the generator is applying microwave energy to the targeted tissue region; (ii) the footswitch 32; (iii) the graphical user interface screen; and (iv) a speaker to generate audible alarms or status sounds. Also communicating with the master controller 58 may be (v) a radio-frequency identification (RFID) reader, which provokes signal transmission from passive RFID tags 158 carried by the applicator-tissue interface 16 or its packaging (see Fig. 25) to identify the applicator-tissue interface 12 prior to use, as will be described later. The RFID reader can also function, if desired, to erase information carried by a RFID tag 158 by a RFID tag 158 after being read, e.g., to prevent reuse of a given application-tissue interface 16 (alternatively, reading and identification functions based upon bar-coded information may be used); and (vi) a sensor 160 on the holster 20 that indicates when an applicator resides on the holster 20.

Regarding the holster 20 (see Fig. 2), the holster 20 is operative for storing the applicator on the system in two secure positions: 1) facing "in," when the applicator-tissue interface 16 carried by the applicator faces toward a power absorber 168 carried by the holster 20, in which the master controller 58 can execute a "test" mode to verify the delivery of power, water temp sensor response, antenna switching where the power is safely contained by the power absorber 168, and 2) facing "out" away from the power absorber 168 for easy access to the attachment point for the applicator-tissue interface 16. When the applicator 14 is in the holster 20, the "facing in" position information desirably verifies that any power delivered during the "test mode" shall be safely contained by the power absorber 168. The facing "in" position is sensed by a magnetic sensor 160 on the holster 20 and a magnet 162 carried by the applicator that registers with the magnetic sensor 160 if and only if the applicator 14 and applicator-tissue interface 16 is facing "in." The "facing out" position need not necessarily be sensed.

Sensed operating conditions are also communicated to the master controller 58. The sensed conditions may include (i) sensed forward power signals detected by the microwave generator 66; (ii) sensed reverse power detected directly by the master controller 58; (iii) sensed negative pressure levels in the vacuum supply line 44, which may be sensed both upstream and downstream of the vacuum solenoid valve 90; (iv) sensed coolant flow in the the coolant supply line 146; (v) coolant level in the coolant reservoir 142; and (vi) sensed temperature of the thermoelectric cooler (TEC 138). Other sensed operating conditions that also may be communicated to the master controller 58 by the applicator main board 60, e.g., (i) sensed forward and reverse power signals detected onboard the applicator 14; (ii) microwave switch status conditions; and (iii) sensed temperature conditions processed by the main applicator board from signals received by the thermocouples 154 residing on the cooling plate 28 and coolant supply line 146 in the applicator.

The master controller 58 also communicates energy generation signals to the microwave generator 66, and operating signals to the solenoid vacuum valve 90. Preprogrammed rules or logic on the master controller 58 process sensed information communicated to the master controller 58 to generate command signals and alarms when an out of bounds condition exists. Based upon the processed information, the master controller 58 may, e.g., increase or decrease fan speeds to maintain the TEC 138 at a desired temperature; increase or decrease coolant flow; or alter power levels.

In a representative embodiment, the master controller 58 desirably is capable of supporting communication and control with the applicator 14. The master controller 58 desirably receives from the applicator information about the applicator temperatures, antenna power and state of the "trigger" switch on the applicator. The master controller 58 desirably sends to the applicator 14 information describing which antennae should be enabled. The master controller 58 desirably sends to the applicator 14 commands to control the applicator LED's, if any. The communication desirably includes fault conditions detected in the applicator. The master controller 58 is desirably capable of supporting the following system states indicated on the applicator: Ready, Treatment, Cooling and Fault.

The master controller 58 desirably serves to detect failures or unexpected/out of tolerance behavior and react to minimize risk of injury to the patient and, when possible, damage to the device.

For example, a prescribed incremental loss of vacuum (e.g., more than 17 kPa (5 inches of Hg vacuum)) may immediately pause the energy delivery cycle. If the incremental loss persists for less than prescribed period of time (e.g., less than 2 seconds), energy delivery may resume when the vacuum level returns to the prescribed level. If the incremental loss persists for longer than the prescribed interval, the master controller 58 may abort the therapy cycle and cause it to enter a post-cool phase.

For example, loss of communications to the applicator or the generator may cause the master controller 58 to enter a safe state by aborting the therapy cycle and causing it to enter the post-cool phase by terminating energy delivery during loss of applicator communications and disabling the amplifier (mute enabled) during loss of amplifier communications.

For example, temperature monitoring at the applicator cooling plate 28 may be used to detect treatment conditions. For example, if the temperature exceeds a predetermined amount (e.g., 40°C) within the first 2 seconds of energy delivery, the master controller 58 may abort the therapy cycle and cause it to enter the post-cool phase. If the temperature exceeds the predetermined amount after the first 2 seconds of energy delivery, the master controller 58 may immediately terminate energy delivery to that antenna and initiate energy delivery to the next antenna in the therapy sequence.

For example, vacuum pump drive may be monitored and compared to nominal drive levels with tolerance bounds to make sure that excessive vacuum leaks that could occur when the target tissue has not been properly acquired or during loss of tissue acquisition are properly reported to the caregiver through the user interface. If the vacuum pump drive fluctuations are excessive during a therapy cycle, the master controller 58 may abort the therapy cycle and cause it to enter the post-cool phase.

For example, internal voltage monitoring of the power supply voltage inside the generator and on the master controller 58 may be used to determine if a fault condition exists that may abort the therapy cycle and cause the system to enter the post-cool phase.

For example, microwave power may be continuously monitored in the console and at the applicator during energy delivery and non energy-delivery phases of the therapy cycle. During energy delivery, the microwave power may be required to be within a specified range and during the non energy-delivery phases of therapy it should be less than the specified threshold. If a fault condition exists, the master controller 58 may abort the therapy cycle and cause the system to enter the post-cool phase. If a fault condition exists during the non energy-delivery phases of therapy, the generator may be disabled (mute enabled).

For example, internal temperatures of critical components may be monitored for detection of excessive thermal conditions.

For example, if there are thermoelectric cooler errors, temperature errors, flow rate errors, or water level errors during a therapy cycle, the master controller 58 may abort the therapy cycle and cause it to enter the post-cool phase.

The master controller 58 may provide the capability to store data associated with each treatment cycle that the system performs. This data may be stored in memory that is not erased when the power to the system is removed or turned off. The information contained in the data log may be made accessible through a service mode screen on the graphical user interface 62. It may store some or all of the following information for each applicator placement in a treatment data log in a folder in system memory: (i) Date and Time; (ii) Average forward power; (iii) Maximum reverse power (from the master controller 58) and/or from each applicator detector board, as will be described below) ; (iv) Temperature rise (delta) for each of the temperature sensors located on the applicator cooling plate 28; (v) Maximum coolant temperature; and/or (vii) Fault Events and associated Error codes.

B. On Board the Applicator

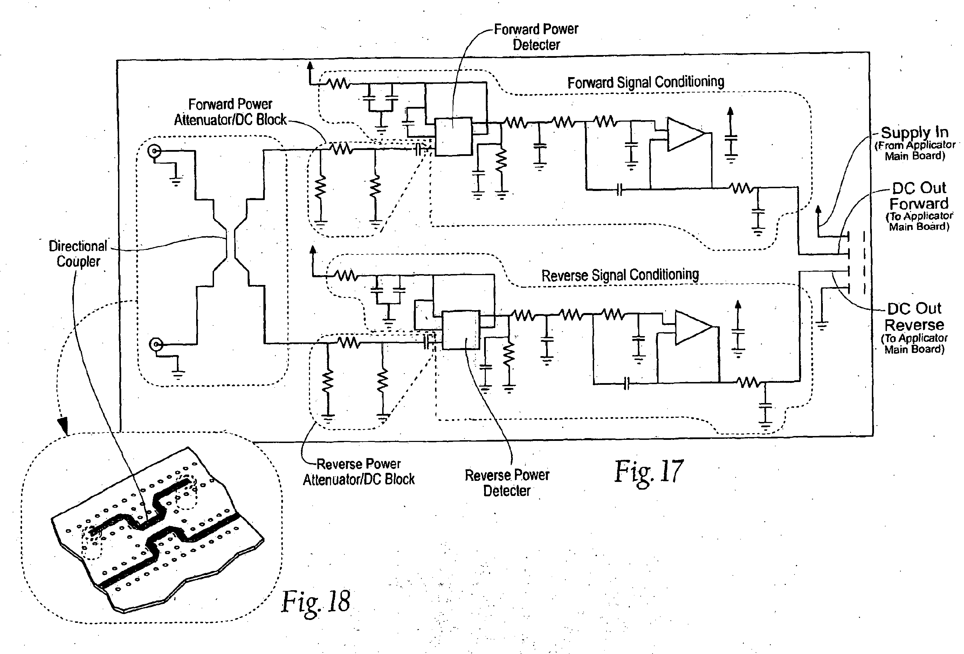

As best shown in Figs. 7 and 8, a microwave switch 26 in the applicator has an input coupled to the microwave energy supply lead. The microwave switch 26 can comprise, e.g., a switch manufactured by Relcom Technologies, Inc., Part No. RMT-SR019. Individual feed connector cables 70 may lead from outputs of the switch 26 to individual detector boards 170, which include directional couplers (see Fig. 17) to pass the microwave signal to the waveguide antennas 24, as well as couple the signal to forward and reverse power detection circuitry.

The master controller 58 on-board the system console 12 includes imbedded pre-programmed rules establishing the desired switching patterns for applying the microwave signal through the waveguide antennas 24. The signals from the master controller 58 are converted by the applicator main board into switching signals which, in turn, control the operation of the switch to execute these patterns.

1. Localized Forward and Reverse Power Detection

As just stated, forward and reverse power signals are detected by the master controller 58 as the microwave signal is transmitted through the special purpose cable assembly to the system applicator 14. These forward and reverse power signals, local to the console 12, are communicated to the master controller 58 for power control purposes.

In addition, the system applicator 14 may carry additional on-board a detector board circuit (see Figs. 17 and 18) for detecting forward and reverse power locally on the applicator. These forward and reverse power signals, local to the applicator 14, are communicated by the applicator main board 60 to the master controller 58 on-board the console 12 for processing as further confirmation that power settings are within prescribed bounds.

As shown in Fig. 7, the detector board circuit comprises four detector boards 170, a given detector being electrically coupled and dedicated to a single waveguide antenna 24. The electrical configuration of each detector board 170 is essentially the same, as will now be described.

(a) Directional Coupler

Fig. 17 shows the circuit traced on each detector board. The circuit includes directional coupler (also shown in Fig. 18) having a thru line and a coupled line (which have been generally described previously). The thru line has an input that receives the microwave signal, as switched to the individual waveguide antenna 24 by the system applicator main board 60, and an output that conveys the received microwave signal to the respective waveguide antenna 24. As best shown in Fig. 18, the coupled line runs in proximity to the thru line for a for a fixed distance, to allow a sample of energy travelling through the thru line to be transferred into the coupled line. The coupled line is directional, meaning that it samples the forward power on one port of the coupled line and the reflected power on the other port. The length of the coupled section, the distance between the thru and coupled lines, the optimization of coaxial feeds to efficiently transfer power, the mountings, and the way in which the ports of the coupler are terminated determine the amount of power sampled in the forward and reflected ports and the isolation between the two ports.

In the illustrated embodiment (see Fig. 18), the directional coupler circuit is implemented using asymmetric stripline transmission lines (a stripline in which the center conductor is not equidistant from the upper and lower ground planes). Energy is fed into the input port and out of the output port through the ground plane which is closer to the center conductor of the stripline. The forward and reverse sampling ports include a transition from asymmetric stripline to microstrip line. This allows for easy placement and assembly of the discrete components of the detector circuit.

(b) Attenuator and DC Blocking Circuit

The forward and reverse sampled power ports feed microwave energy into a microwave attenuator and DC blocking circuit as shown in Fig. 17. The microwave attenuator circuit serves to condition the amount of microwave power to an optimal range of levels for the power detector. The circuit also includes a DC block such that no low-frequency signals can travel from the power detector or signal conditioning circuitry down the high frequency microwave path.

(c) Power Detector

The circuit (see Fig. 17) includes a reverse power detector and a forward power detector. The power detectors convert the high-frequency microwave signal to a low-frequency AC or DC signal. The converted low-frequency signal has a strength that is directly proportional to or indicative of the strength of the input high-frequency signal. The detectors can comprise, e.g., an ADL-5510 Detector IC (available from Analog Devices), or another active/passive detection device.

(d) Signal Conditioning Circuitry

The circuit (see Fig. 17) includes signal conditioning circuitry for each forward and reverse power detector. The signal conditioning circuitry performs three main functions. It provides a clean (non-noisy) supply voltage to the power detector and any other active components. It filters out any unwanted internal or external noise in the detector circuit. Finally, it serves as a low-pass filter for the low-frequency output of the power detector. This reduces any low-frequency AC signal coming out of the power detector (i.e. as a result of a pulse-width modulated microwave signals) to a DC signal for subsequent digitization on the applicator main board and transmission back to the master controller 58 onboard the console 12.

2. Temperature Sensing

The temperature condition measurements from the thermocouples 154 along the cooling paths and at the tissue cooling plate 28 are also communicated by the system applicator main board to the master controller 58 of the system console 12 via the connection links 156 through the special purpose cable assembly 34. Based upon this closed loop feedback, the logic residing in the master controller 58 may control power as part of the energy generation function.

3. The LED Indicator Board

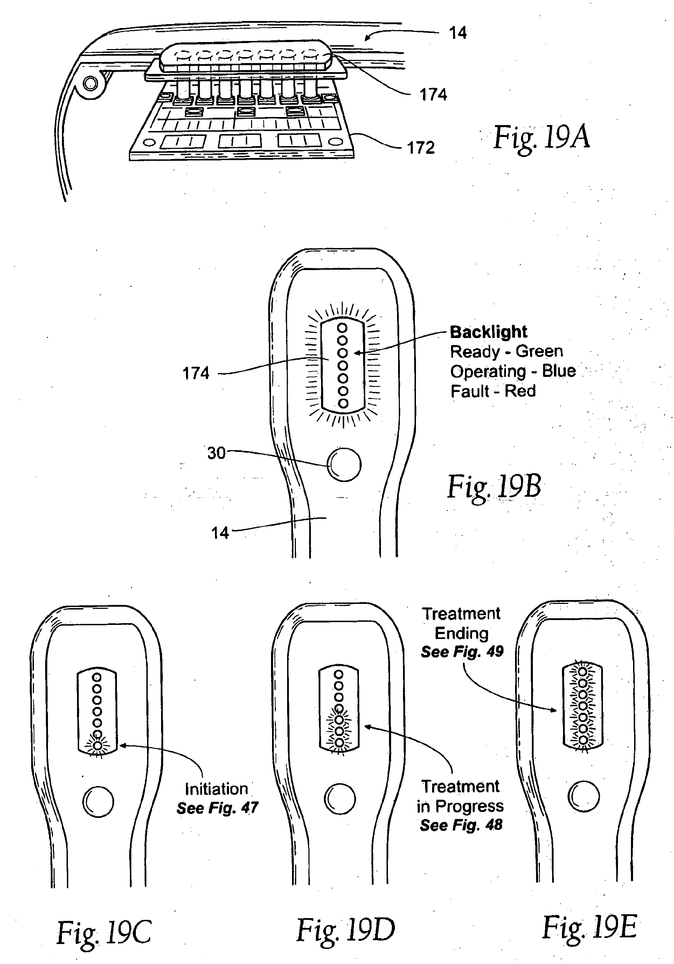

In the illustrated embodiment (see. Figs. 7 and 19A/B/C/D), the LED indicator board 172 includes an appropriate number of LED's and/or lightpipes 174 that are arranged on a display area visible to the caregiver on the housing adjacent the switch 30.

In a representative embodiment, there are 15 LED's and 7 lightpipes. The system applicator main board 60 communicates sensed status and operational information from the master controller 58 to the LED indicator board 172. The LED indicator board 172, in turn, commands operation the LED's and/or lightpipes 174 according to pre-programmed rules in term of the display of colors and/or light patterns and/or backlighting colors and patterns, to visually communicate these status and operational conditions to the caregiver.

The presence of desired operating conditions and out of bounds conditions can be visually represented by different backlighting colors, as can the status of a treatment cycle. The nature and content of information visually communicated by the LED indicator board can be widely varied and tailored to the needs of the individual system 10.

For example, LED's can be backlighted indicating the status of operations (see Fig. 19B); for example, a green backlight may indicate a system ready condition; a blue backlight may indicate when energy is being applied to tissue; and a red backlight may indicate a fault or error condition. LED's may be sequentially illuminated to indicate the status of treatment, e.g., a single LED to indicate the initiation of treatment (see Fig. 19C), all LED's illuminated indicating the end of treatment (see Fig. 19E); and intermediate numbers of LED's being illuminated as treatment proceeds (see Fig. 19D).

C. Conclusion

In the representative embodiments, decision making function of the system applicator main board 60 may be extensive or purposely limited. In the illustrated embodiment, the pre-programmed rules residing on the system applicator main board 60 may switch the microwave switch 26 and LED's 174. The system applicator main board 60 may communicate to the master controller 58 any detected error, the status of the power switch on the system applicator 14, LED's and microwave switch 26 positions, sensed temperature conditions, and measured forward and reverse power. The master controller 58 on-board the system console 12 may make all other control decisions based upon these data.

III. Use of the System

A. Anatomy of the Skin

Fig. 20 shows an idealized and simplified anatomic schematic drawing of the human skin - the body's largest organ -- and its structures. Fig. 20 shows in idealized and simplified form the layered arrangement of the body's covering and the hairs and glands embedded within the skin and subcutaneous tissue.

The skin consists of the epidermis (a superficial cellular layer) and the dermis (a deeper connective tissue layer). The subcutaneous tissue below the dermis (the hypodermis) is composed of loose, fatty connective tissue. Located between the dermis and the underlying deep fascia, the hypodermis contains hair follicles, sweat glands, blood vessels, lymphatics, and cutaneous nerves.

As shown in idealized and simplified form in Fig. 20, the interface between the dermis and the hypodermis typically is non-linear and non continuous, comprising an irregular interface, which may also include many tissue structures or groups of tissue structures which cross and interrupt the tissue interface.

The deep fascia is a dense, organized connective tissue layer bellow the hypodermis that invests deep structures such as the muscles.

The deep dermis and hypodermis may contain hair follicles with their associated smooth arrector pili muscles (which contact to cause "goose bumps") and sebaceous glands (which, when compressed by contraction of the arrector pili muscles, express their oily secretion onto the skin surface).

The deep dermis and hypodermis may also contain a larger number of sweat glands. Apocrine sweat glands produce a complex secretion that may generate a strong odor and are numerous in certain areas of the body, such as under the arms and in the genital region. Eccrine sweat glands are also generally distributed throughout the entire hypodermis, and are numerous in the palms of the hands, soles of the feet, and axilla.

The sweat glands produce perspiration in response to stimuli, including emotional stimulation and to adjust body temperature. Some people sweat more in warm temperatures, when they exercise, or in response to situations that make them nervous, angry, embarrassed, or afraid.

B. Application of Microwave Energy to the

Skin Using the System

In use, the system 10 may apply microwave energy to the skin, e.g., to treat hyperhidrosis. To set up for use (as Fig. 1 generally shows), an unused applicator-tissue interface 16 is joined to the system applicator 14. The vacuum supply conduit 44 can be carried in a recess in the pistol grip of the system applicator 14, to run along the special purpose cable assembly 34 for coupling to a vacuum connection port 48 on the system console 12. The special purpose cable assembly 34 is likewise coupled to the special purpose connector 38 on the system console 12, coupling the system applicator 14 to the system console 12. The main power switch of the system console 12 is system console 12 is turned on, and the master controller 58 of the system console 12 executes a start up and initialization routine. The caregiver sets the commanded power and vacuum conditions. The master controller 58 initiates a constant circulation of coolant through the system applicator 14.

After identifying the tissue region to be treated, the caregiver places the compliant skirt 106 of the interface body against skin in the targeted tissue region (as shown in Fig. 21A). The edges of the compliant skirt 106 form a seal against the skin. The caregiver actuates the external power switch on the housing. The system console 12 supplies negative pressure through the vacuum supply conduit 44 into the chamber 42. The negative pressure in the chamber 42 serves to draw tissue into the chamber 42 (i.e., elevate the tissue) into contact with the applicator-tissue interface surface 100/52 in thermal contact with a least a portion of the cooling plate 28 (as shown in Fig. 21B). The vacuum within the tissue acquisition chamber 42 elevates the dermis and hypodermis, separating dermis and hypodermis from muscle. The vacuum within the tissue acquisition chamber 42 localizes and stabilizes the tissue region within the chamber 42. By separating the dermis and hypodermis from muscle, the vacuum within the tissue acquisition chamber 42 serves to protect muscle by limiting or eliminating the electromagnetic energy that reaches muscle. The vacuum is applied until a desired vacuum condition and/or tissue temperature condition is achieved. Alternatively, a delay period for a prescribed interval of time can occur.

Once the desired vacuum and/or temperature condition is sensed (or after a prescribed delay, if relied upon); the system console 12 supplies the microwave signal to the system applicator 14. The microwave signal generated by by the system console 12 is applied in a predetermined manner to the tissue region. Its electromagnetic radiation may be radiated at a frequency of, for example, between 5 and 6.5 GHz, or at a frequency within that range of about 5.8 GHz, at a power entering an individual antenna of between 20 to 60 W, desirable between 25 and 45 W, more desirably between 32 to 38 W, and most desirably 35 W. It should be appreciated that, if power is measured leaving the generator, the power magnitudes expressed above will be greater due to power loses through cables and other power losses between the generator and the antenna. For a relatively short cable, a power of 55W measured at the generator will likely yield the desired power range at the antenna. For longer cables, the power measured at the generator must be increased (e.g., up to 65 W) to achieve the desired range of power levels at the antenna.

The microwave power may be applied in succession to individual antenna, e.g., in the progression antenna A, then antenna B, then antenna C, and then antenna D, or antenna A, then antenna C, then antenna B, and then antenna D. The microwave may be applied in prescribed time increments at each antenna, e.g., antenna A (3 seconds), antenna C (3 seconds), antenna B (3 seconds), and antenna D (3 seconds), followed by a post-cooling interval based upon time (e.g., 20 seconds), then followed by a release of vacuum pressure.

The microwave power may, alternatively, be applied to both individual antennas and split between pairs of antennas in phase drive mode, as will be described in greater detail later. The sequence can comprise, e.g., antenna A (2.5 seconds), then antennas A-B (2.5 seconds); then antenna B (2.5 seconds), then antenna B-C (2.5 seconds), and so on, followed by a post-cooling interval based upon time (e.g., 20 seconds), then followed by a release of vacuum pressure.

C. Creation of Lesion Patterns

Components carried on-board the system console 12 (see Fig. 15) apply the microwave signal through the waveguide antennas 24 to tissue acquired within the tissue acquisition chamber 42 to carry out the lesion creation function.

The microwave signal may be applied, for example, in succession by each waveguide antenna. Alternatively, the microwave signal may be applied in succession by a single waveguide antenna (A), then concurrently by the single waveguide antenna and the next adjacent waveguide antenna (AB) with the microwave power applied to adjacent antennas 24 in phase (i.e., such that the energy applied results in constructive wave interference between the radiated energy from each antenna at the targeted region), and then by the next adjacent waveguide antenna (B) alone, and then by the the waveguide antenna (B) and its next adjacent antenna (C) (i.e., BC) with the microwave power applied to adjacent antennas 24 in phase (i.e., such that the energy applied results in constructive wave interference between the radiated energy from each antenna at the targeted region), and so on in succession C-CD-D until all waveguide antennas 24 have been involved (which in shorthand is called a phase-driven mode).

When pairs of antennas are switched to simultaneously radiate energy, field interference patterns are created due to two phenomena: (i) "standing wave" interaction between forward travelling waves propagating through the epidermis/dermis and reverse travelling waves reflected off of the dermal/hypodermal boundary, as well as (ii) "phase" interaction between the signals radiated by each antenna.

First, interactions occur when energy radiated by the two antennas propagates through the epidermis/dermis and then reflects off of the dermal/hypodermal interface back into the dermis. A standing wave pattern is created where the forward and reflected signals generate an interference pattern that varies primarily in the direction perpendicular to the tissue planes (i.e. varies with depth in tissue). The wavelength (and correspondingly the frequency) of the radiated signal determines the regions in which the standing wave pattern is constructive and destructive. An "optimal standing wave interference pattern" is created by choosing a frequency (e.g. 5.8GHz) such that constructive interference occurs in the deep dermal region and is minimized in the shallower dermal and epidermal region.

Second, interactions occur when energy radiated by each antenna interfere with each other. An antenna interference pattern is created where the differences in the phase of the radiated energy from each antenna determine regions where the individually radiated signals add constructively or destructively. The variation of the interference pattern with phase occurs primarily in the direction parallel to the tissue planes. An "optimal antenna interference pattern" is created by choosing a phase relationship between the two antennas such that constructive interference occurs in the region between the two antennas and destructive interference occurs in the region underneath each individual antenna. A phase difference of 0 degrees (i.e. "in-phase") between radiated signals from the antennas is the optimal phase relationship for achieving the "optimal antenna interference pattern."

To achieve this phase relationship, phase-balanced interconnecting cables can be utilized to connect antennas with the same feed direction (e.g. antennas A and B). Similarly, interconnecting cables with a 180 degree phase degree phase difference should be utilized to connect antennas with opposite feed directions (e.g. antennas B and C).

In phase-driven mode, when two antennas radiate energy concurrently, the energy has the same frequency and the antennas are driven in phase. The power provided from the generator to the microwave switch is split between the two antennas such that each radiates on-half of the supplied power. The overall interference pattern in tissue is optimal in terms of both the standing wave interference pattern and the antenna interference pattern. This occurs since the two interference phenomena are largely independent, with the standing wave interference occurring in the perpendicular direction and the antenna interference occurring in the parallel direction. As a result, an overall interference pattern that is constructive in the deep dermal region between the two antennas and is destructive in the shallow dermal/epidermal region and in the region underneath individual antennas is achieved.

If applied by an individual waveguide antenna at a given frequency and power level (see Fig, 22A), a first region of peak tissue effect initiates generally beneath the scattering element 78 of the waveguide antenna and in the deep dermis (above the interface between the dermis and hypodermis). This is the result of the "optimal standing wave pattern' creating a maximum power absorption in the deep dermis in the region where constructive interference occurs.

If applied concurrently through two adjacent waveguide antennas 24 at the same frequency and in phase, and at a total power from the generator that is split equally between the antennas 24 (see Fig. 23A), a first region of peak tissue effect initiates generally between the scattering elements 78 of the waveguide antennas 24 above the interface between the dermis and hypodermis. This constitutes the desired concurrent effect of both standing wave interference pattern and antenna interference pattern. The standing wave interference pattern initiates in the region of peak tissue temperature generally above the interface between the dermis and hypodermis, just as when energy of the same frequency is applied by an individual waveguide antenna. The antenna interference pattern initiates in the region of the first region of peak tissue effect generally within the same tissue plane, but between the scattering elements 78 of the waveguide antennas 24 and at generally the same power level as when energy is applied by an individual waveguide antenna (the power that has been divided in half and fed into each antenna 24 is recombined in tissue).

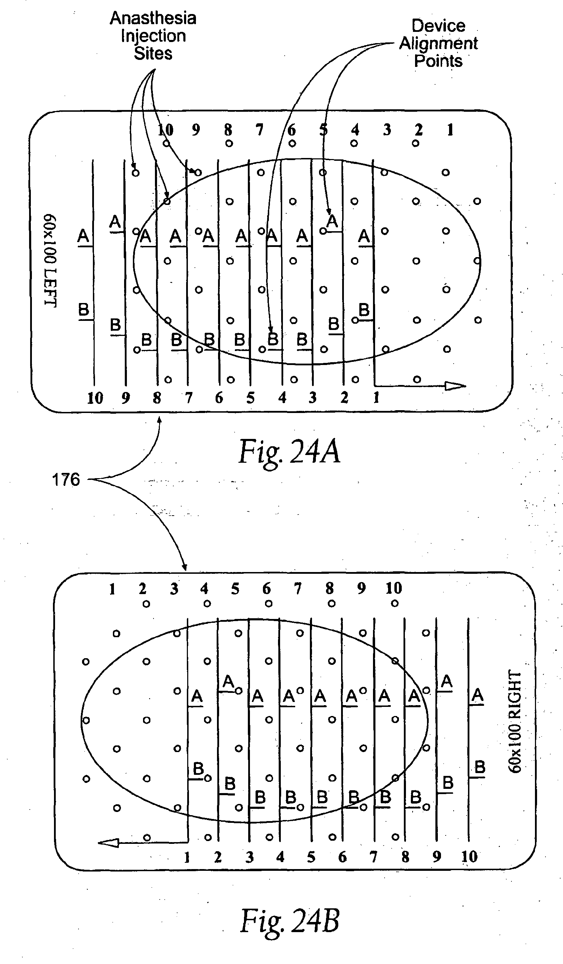

The peak tissue effect can be expressed, e.g., in terms of peak Specific Absorption Rate (SAR), which is a measure of the rate at which the energy is absorbed by tissue (in terms of power absorbed per mass of tissue in units of watts per kilogram). Alternatively, the peak tissue effect can be expressed as, e.g., peak power loss density, or a peak tissue temperature. (as Figs. 24A and 25A show), the first region of peak tissue effect initiates in the dermis generally above the interface between the dermis and hypodermis, due to a standing wave effect the interface imposes upon the microwave signal in the dermis. The interface reflects electromagnetic waves radiated by the waveguide antenna to cause constructive wave interference above the interface, initiating the peak tissue effect.

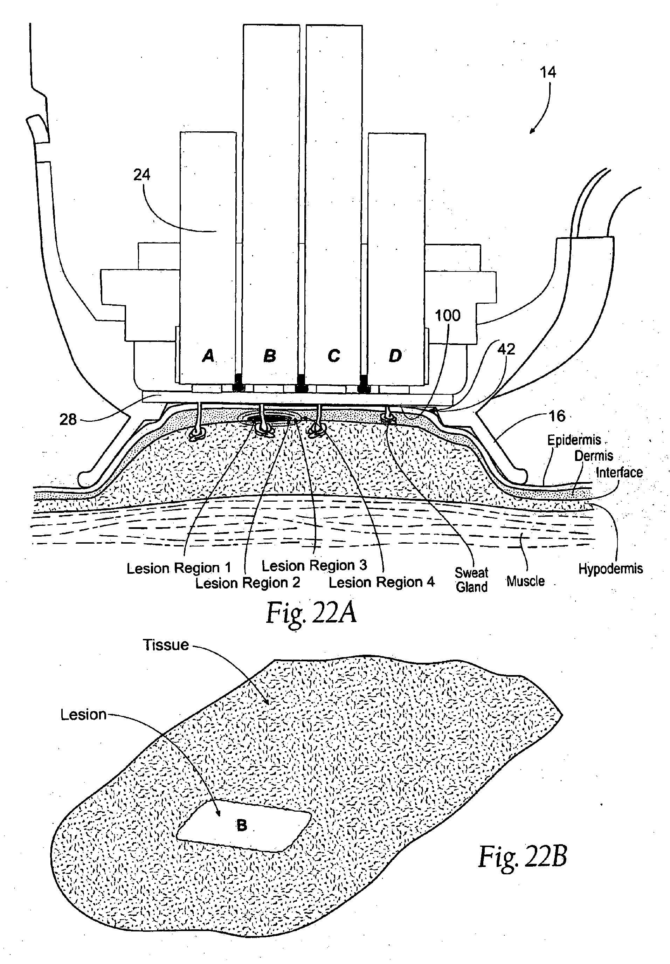

As Figs. 22A and 23A show, successive second, third, and fourth regions of tissue effects are observed to spread from the first region due to conductive/convective heating effects with reduced tissue effect magnitudes at increasing radial distances from the first region. These "ripple" regions of diminishing tissue effects extend toward the epidermis and, in part, can extend below the interface into the hypodermis, as Figs. 22A and 23A show.

The tissue effects serve to create a localized lesion in the first tissue region within the dermis (see Figs. 22B and 23B). The localized heating effect in the dermis can, by resulting conductive/convective heating effects, damage or destroy structures in the dermis and/or hypodermis, such as, for example, sweat glands in the skin of an individual undergoing treatment.

The scattering element 78 and intermediate scattering element 80 may be used, for example, to spread and flatten the first region of peak tissue effect in terms of peak SAR, and/or peak power loss density, and/or peak tissue temperature. The scattering element 78 and intermediate scattering elements 80 can thereby serve to spread and flatten the lesion formed in first tissue region to further control the localized effects. The temperature conditions established by the cooling plate 28 keep the lesion from expanding toward the epidermis.

By programming the master controller 58 to switch the waveguide antennas 24 in a predetermined pattern, the microwave signal generated by the system console 12 can be applied to the skin to form complex patterns of lesions. For example, as shown in Fig. 23B, lesions may be created in a predetermined order, such as, for example A-B-C-D, where: A represents a lesion initiated directly under waveguide antenna A; B represents a lesion initiated directly under waveguide antenna B; C represents a lesion initiated directly under waveguide antenna C; and D represents a lesion initiated directly under waveguide antenna D. Overlapping lesions can be formed in the tissue intervals between lesions A-B-C-D by creating lesions in a predetermined order, for example, A-AB-B-BC-A-AB-B-BC-C-CD-D where: A represents a lesion initiated directly under waveguide antenna A; AB represents a lesion initiated under the intersection between waveguide antenna A and waveguide antenna B; B represents a lesion initiated directly under waveguide antenna B; BC represents a lesion initiated under the intersection between waveguide antenna B and waveguide antenna C; C represents a lesion initiated directly under waveguide antenna C; CD represents a lesion initiated under the intersection between waveguide antenna C and waveguide antenna D; and D represents a lesion initiated directly under waveguide antenna D. A lesion AB may be created between waveguide antenna A and waveguide antenna B, by driving waveguide antenna A and waveguide antenna B simultaneously in phase and with a balanced output from each antenna. A lesion BC may be created between waveguide antenna B and waveguide antenna C, by driving waveguide antenna B and waveguide antenna C simultaneously in phase and with a balanced output from each waveguide antenna. A lesion CD may be created between waveguide antenna C and waveguide antenna D, by driving waveguide antenna C and waveguide antenna D simultaneously in phase and with a balanced output from each waveguide antenna.

It should be appreciated that power can be applied homogenously, with the same power and time increments for each antenna or each pair of antennas 24 (in phase drive mode). Power can also be applied differently among different antennas 24 or pairs of antennas 24. Power can be changed for different antennas 24 or pairs of antennas 24, and/or time can be varied for different antennas 24 or pairs of antennas 24. Thus, the energy delivered to a given tissue region (energy being the product of power and time) can be varied from tissue region to tissue region being treated.

1. The Treatment Template

The system 10 may further include a treatment template 176 (see Figs. 23A and 23B) to provide guidance and placement information for system applicator 14 in a matrix format. The treatment template 176 is sized and configured to overlay an entire axilla (underarm) tissue region targeted for treatment. The template 176 can comprise a temporary tattoo applied to each underarm (left side as shown in Fig. 24A and right side and shown in Fig. 24B). Alternatively, the template 176 can comprise a pattern applied by stamping on a tissue region. The template 176 can comprise an overlay stencil placed on the skin surface and applied by a marker pen through the stencil. The template 176 can comprise an overlay mesh sticker applied to the tissue region.

A family of templates 176 (see Fig. 25) can be provided, with different sizes and arrays, to accommodate the different anatomies of individuals.

The template 176 may include prescribed anesthesia injection sites (small thru holes) to identify appropriate points in the axilla for the injection of anesthesia; and device alignment points in an x-y matrix axis (1A to 10A and 1B to 10B, and more depending upon the size of the axilla) to be used in conjunction with alignment members 108 on the compliant skirt 106 to provide a positioning point of reference to the caregiver during use of the template 176.

IV. Instructions for Use

As Fig. 25 shows, the system applicator 14 and/or applicator-tissue interface 16 of the system 10 can be provided for use in sterile kits 180. In the illustrated embodiment, each kit 180 includes an interior tray 182 made, e.g., from die cut cardboard, plastic sheet, or thermo-formed plastic material. The system applicator 14 and applicator-tissue interface 16 is carried by a respective tray 182. Either kit 180 can also include in the tray or separately packaged a treatment template or family of templates 176.

Each tray 182 may include a tear-away overwrap, to peripherally seal the tray from contact with the outside environment. Each kit 182 carrying the system applicator 14 and/or applicator-tissue interface 16 may be sterilized by convention ethylene oxide (ETO) sterilization techniques. In the illustrated embodiment, the packaging for one or both the system applicator 14 and/or applicator-tissue interface 16 can carry passive RFID tags 158 that interact with radio-frequency identification (RFID) source on the console 12 (shown in Fig. 15).

In the illustrated embodiment, one or both kits 180 also preferably include directions or instructions for using 184 the system applicator 14 and applicator-tissue interface 16 in conjunction with the system console 12 to carry out a desired procedure. Exemplary directions will be described later. The directions or instructions 184 can, of course vary, according to the particularities of the desired procedure. Furthermore, the directions or instructions 184 need not be physically present in the kit. The directions or instructions 184 can be embodied in separate instruction manuals, or in video or audio tapes, or in electronic form. The instructions or directions can also be incorporated into a grapihical user interface, as will be demonstrated later.

Representative instructions 184 direct use the applicator-tissue interface 16 in concert with the system applicator 14 and system console 12 to apply microwave energy to the skin, e.g., to treat hyperhidrosis. These instructions 184 can also be reflected on the graphical user interface 62, as will now be described.

V. Graphical User Interface

The master controller 58 of the system console 12 can includes circuitry to implement a graphical user interface 62 on the display screen 64, as generally shown in Fig. 11. The graphical user interface 62 can provide control and alarm conditions to the caregiver, and allow for touch-screen interaction and input from the caregiver to the master controller 58.

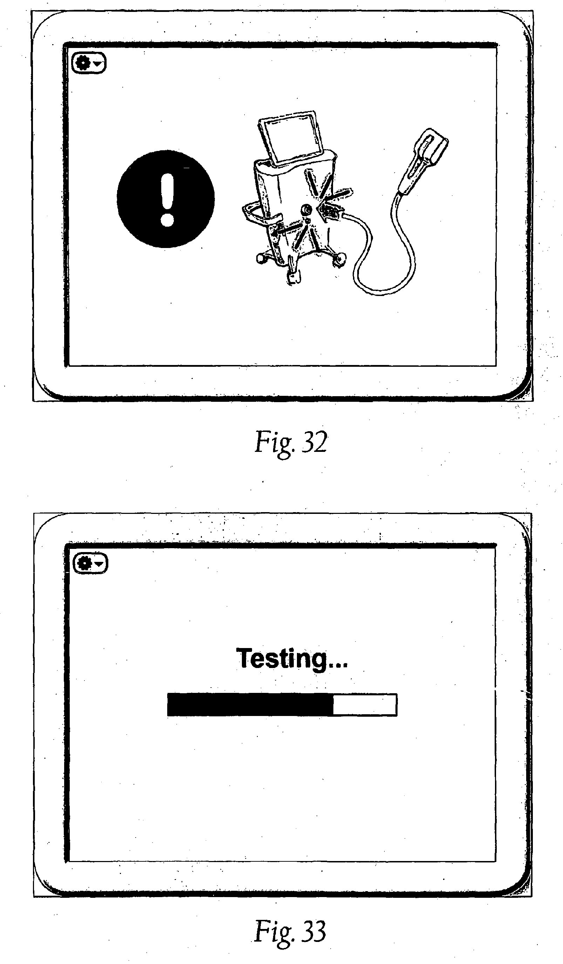

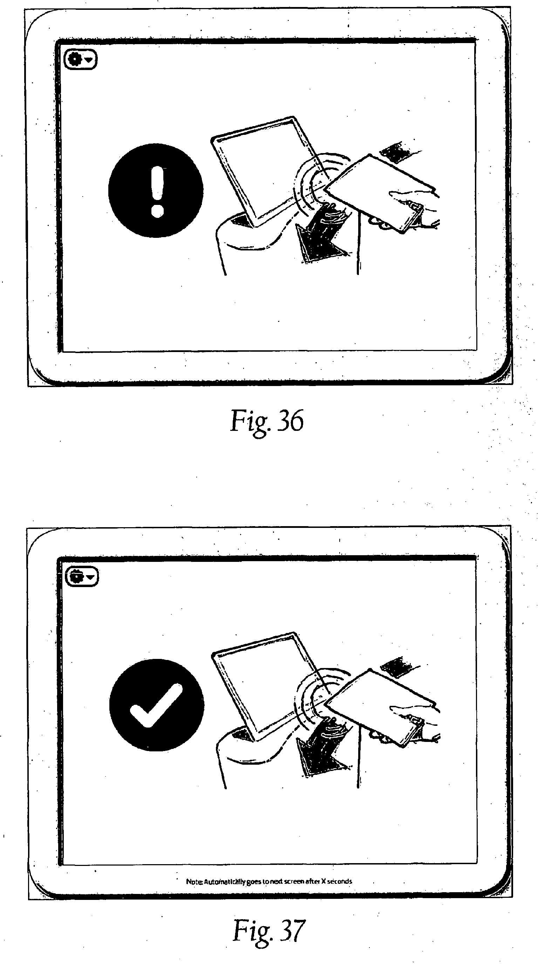

A representative screen for a graphical user interface 62 is shown in Fig. 26. The logic and flow of a representative graphical user interface 62 are shown schematically in Figs. 37 to 31 with reference to representative graphical screen prompts in Figs. 32 to 59.

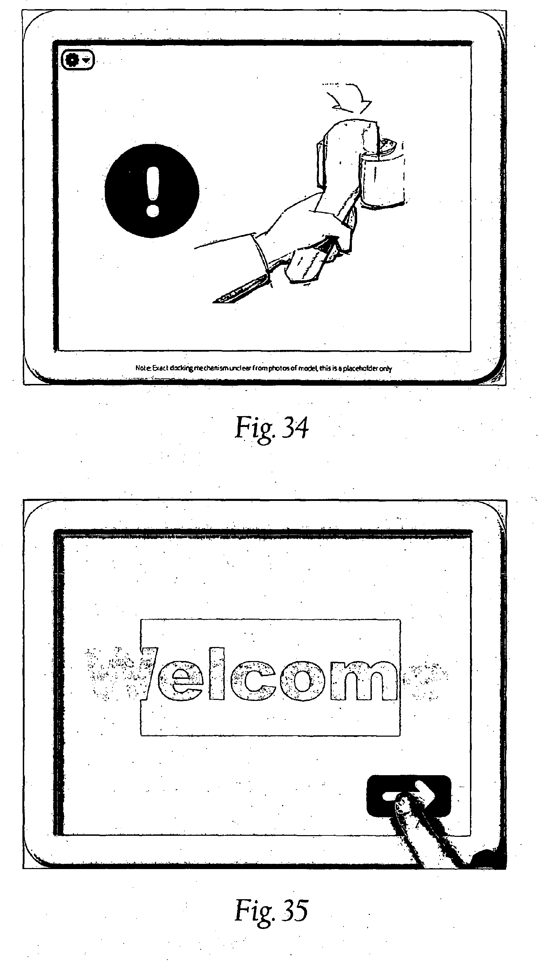

As Fig. 27 shows, the logic and flow of the graphical user interface 62 begins with a start-up routine after power to console is turned on. The master controller 58 determines whether the special purpose cable is plugged in (see prompt in Fig. 32) and then proceeds through a self-test routine (see Fig. 33). If the applicator is not facing "in" on the holster 20, the caregiver is instructed to position the applicator correctly (see Fig. 34). A welcome screen confirms that no errors are detected (see Fig. 35).

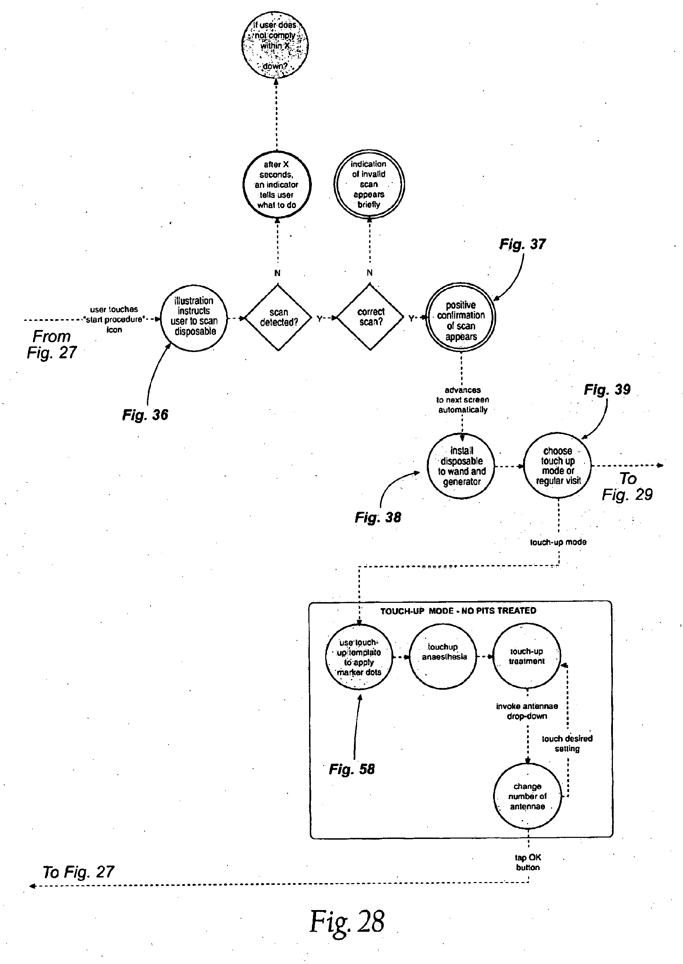

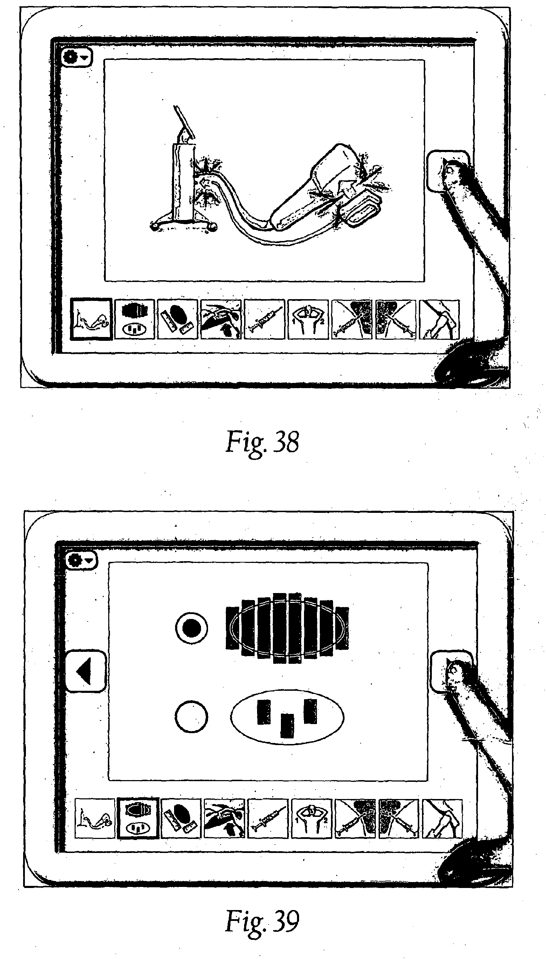

As Fig. 28 shows, the logic and flow of the graphical user interface 62 next instructs placement of the applicator-tissue interface on the applicator (see Fig. 36). The caregiver is instructed to scan the RFID tag 158 on the packaging (see Fig. 25). If the scan confirms that the applicator-tissue interface 16 is approved for use (see Fig. 37), the caregiver is instructed to properly place the applicator 14 with the applicator-tissue interface 16 attached in the facing "in" position on the holster 20 (see Fig. 38) (with the applicator-tissue interface facing the absorber on the holster 20.

Regarding RFID communication, the master controller 58 desirably conditions the RFID reader to detect that an appropriate applicator-tissue interface is being used with the system 10 and to detect, e.g., reuse, if the applicator-tissue interface 16 is intended to be a disposable, single use component. The master controller 58 desirably includes the ability to read secure and encrypted RFID tags 158 attached to the applicator-tissue interface packaging (as Fig. 25 shows), which shall include authorization for a single treatment session (either full treatment or touch-up) and shall be marked as "used" with the date and time of the start of the authorized treatment session. The master controller 58 may retain enough information to restore a guided treatment session to the last placement not fully completed on interruption of the treatment session or loss of power, or to restore the exposure count for a "touch-up" treatment. If it has been longer than, e.g., a 4 hour expiration time for the applicator-tissue interface that is intended to be disposable, resumption of the treatment session is not allowed until an "un-used" disposable RFID tag is read and marked as "used". The master controller 58 may include a "Disposable History" display, listing the date/time a disposable was marked as "used", for the previous 200 disposables, as a minimum. If a "used" disposable RFID tag is read, the date and time the tag was marked as used will be displayed.

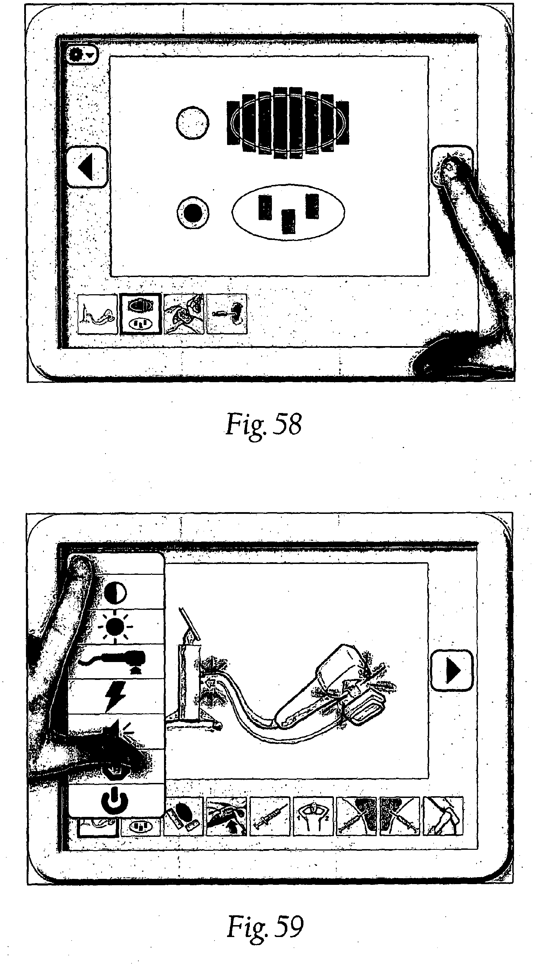

The caregiver is then instructed to choose the mode of treatment - regular or touch up (see Fig. 39). Caregiver choices are communicated by touch screen inputs marked by self-apparent, intuitive icons. If touch up mode is selected before the regular mode (meaning that there has been no prior treatment applied to the targeted tissue region), the caregiver is instructed to proceed with with treatment in the touch up mode (see Fig. 58). The touch up mode in this instance allows the caregiver to directly control the selection of antennas 24 for treatment.

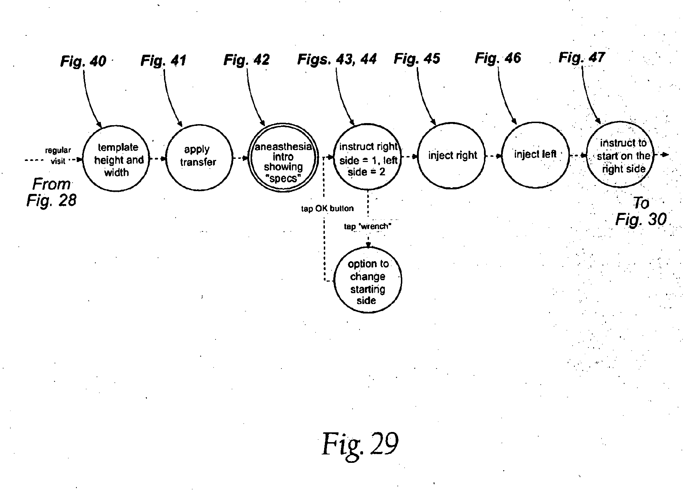

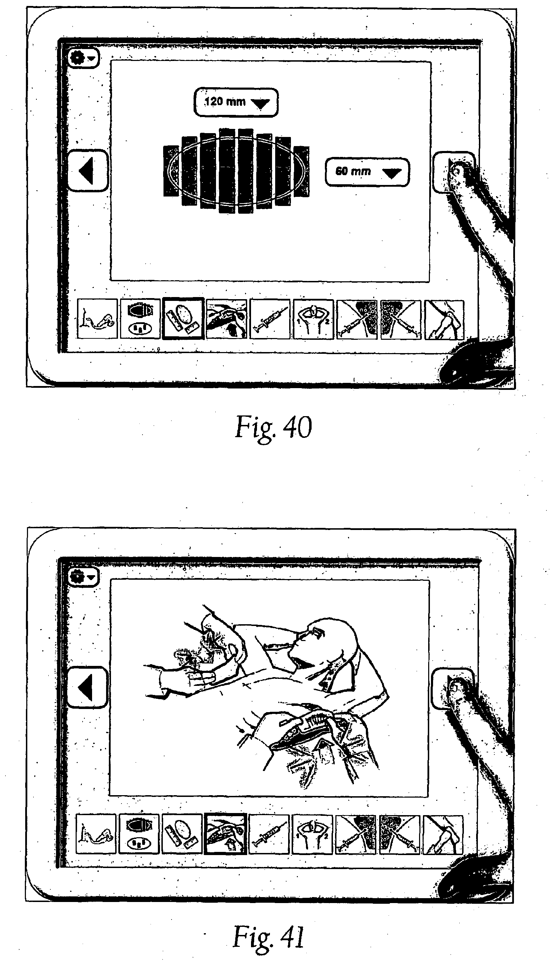

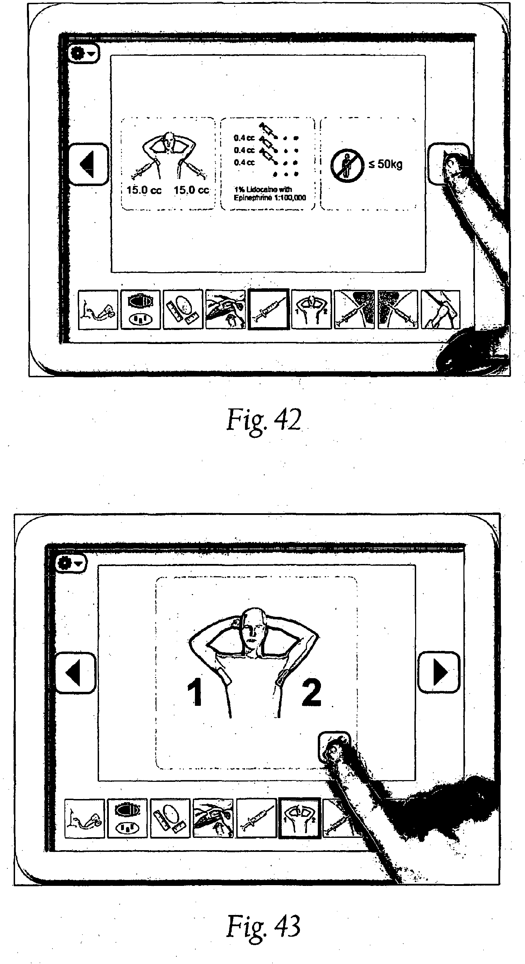

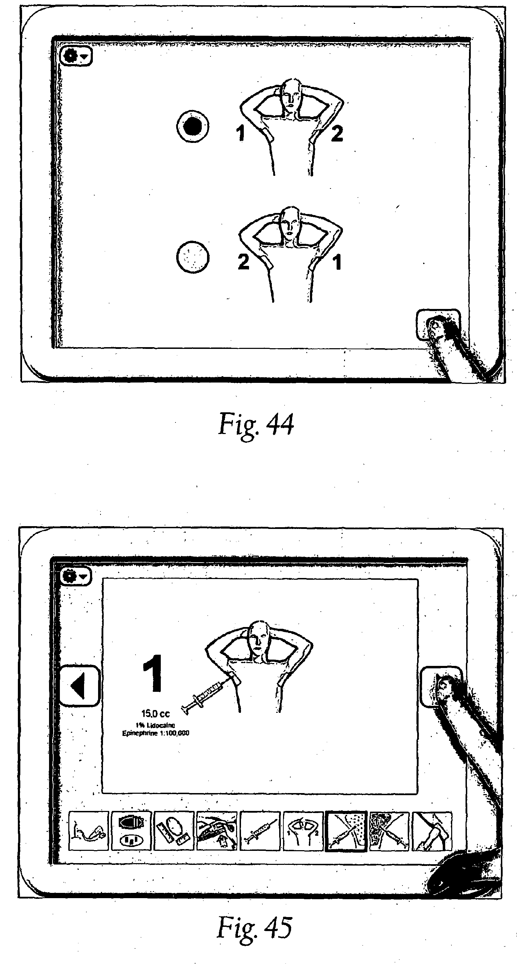

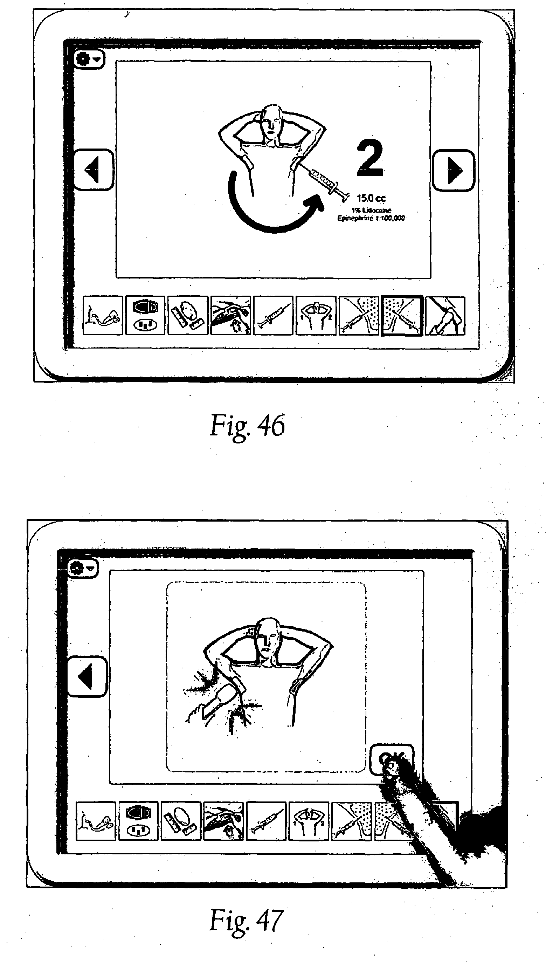

If regular mode is selected (see Fig. 29), the caregiver is guided through a treatment routine. In preparation, the caregiver is instructed to enter the height and weight of the individual to be treated (see Fig. 40); to apply the template 176 (the transfer) (see Fig. 41); to plan on applying anesthesia in.a recommended total amount and in recommended individual aliquots (see Fig. 42); and to select to treat left armpit first and the right armpit second, or vice versa (see Figs. 43 and 44). The caregiver is then instructed to apply the anesthesia (guided by the template) to the first side selected (in Fig. 45, it is the right side first), and then apply anesthesia to the second side selected (in Fig. 46, it is the left side). The caregiver is then instructed to begin treatment on the selected right side first (see Fig. 47).

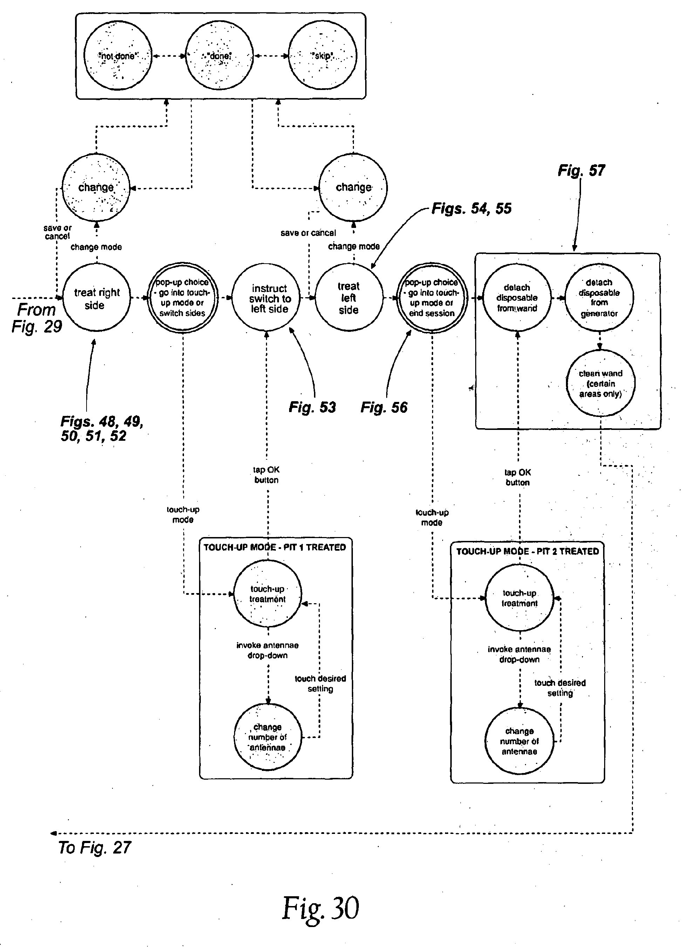

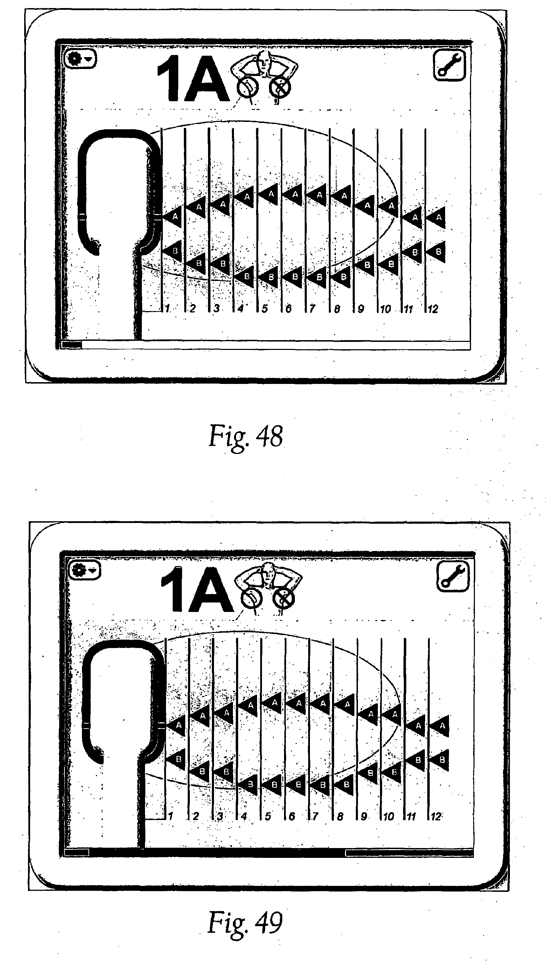

As Fig. 30 shows, the caregiver is instructed through the treatment routine, guided by the template (see Figs. 48, 49, 50, 51, and 52). Guided by the template, the caregiver systematically proceeds by sections (1 to 12) and by regions (A and B) within each section to activate the waveguide antenna array 22 under the control of the master controller 58. The phase drive sequence as described above is repeated at each region for each section to lay down a pattern of lesions at each region-section.

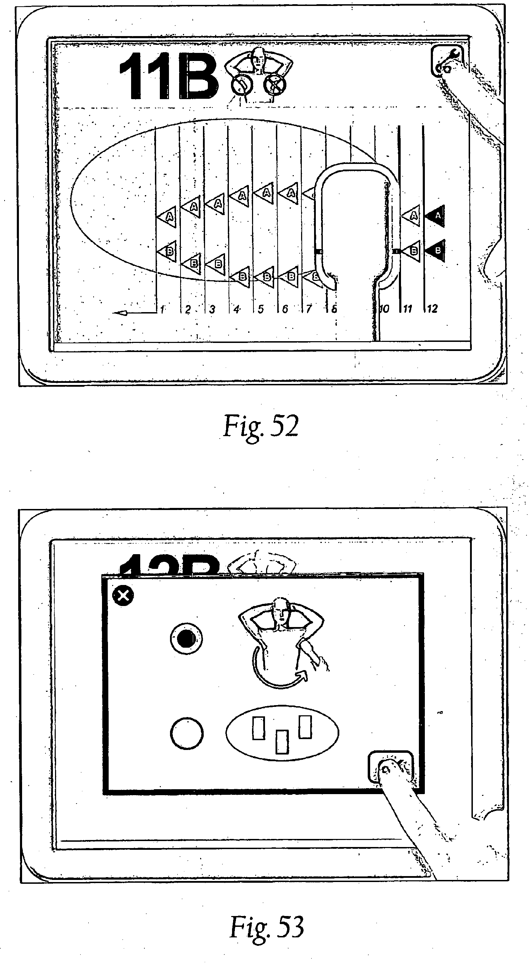

When the treatment routine is completed in one side, the caregiver is asked whether it wants to proceed to the next side, or touch up the same side. During touch up, the caregiver can return to correct lesion formation inconsistencies or gaps. Once touch up is completed on that side (if selected), the caregiver is prompted to switch to the next side (see Fig. 53, where the left side is selected).

The caregiver is then instructed (see Fig. 53) to choose the mode of treatment for the second selected side - regular or touch up, as before described with respect to the first selected side. If touch up mode is selected before the regular mode (meaning that there has been no prior treatment applied to the targeted tissue region on that selected side), the caregiver is instructed to proceed with treatment in the touch up mode. The touch up mode in this instance allows the caregiver to directly control the selection of antennas 24 for treatment on that selected side.

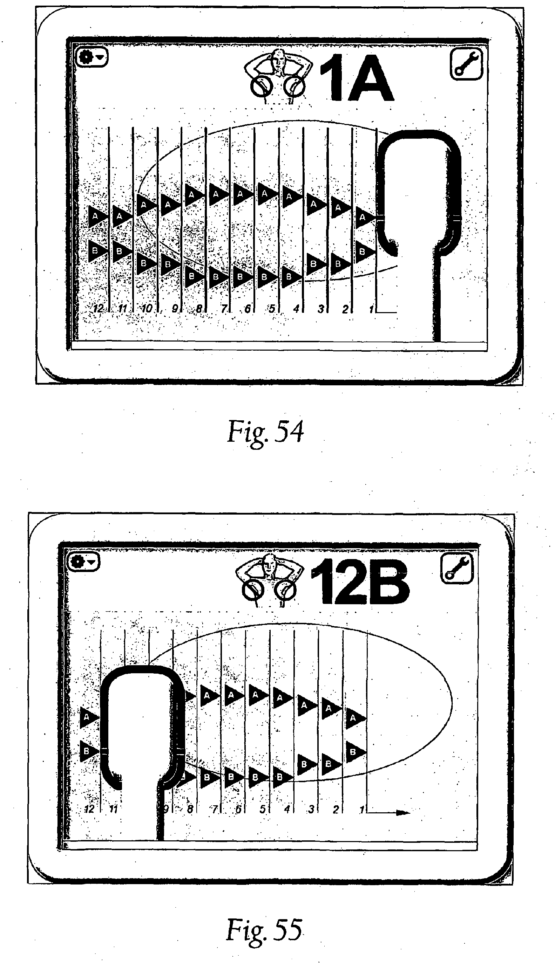

If regular mode is selected for the.second side (as Fig. 53 shows), the caregiver the caregiver is instructed through the treatment routine, guided by the template (see Figs. 54 and 55). Guided by the template, the caregiver systematically proceeds to treat the second selected side by sections (1 to 12) and by regions (A and B) within each section to activate the waveguide antenna array 22 under the control of the master controller 58. The phase drive sequence as described above is repeated at each region for each section to lay down a pattern of lesions at each region-section.

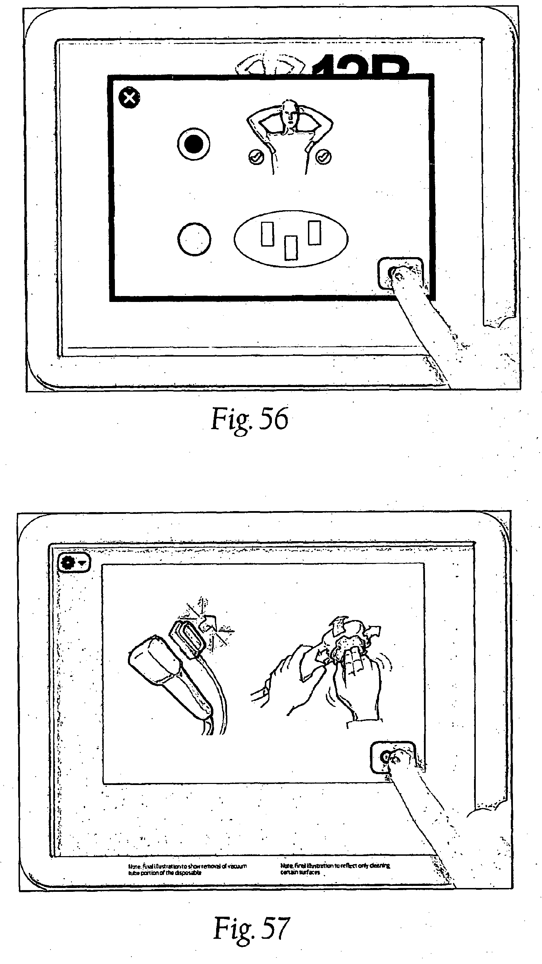

When the treatment routine is completed in the second side, the caregiver is asked whether it wants to end the session or touch up the just completed side (see Fig. 56). During touch up (see Fig. 58), the caregiver can return to correct lesion formation inconsistencies or gaps. Once touch up is completed on that side (if selected), or if the caregiver has selected to end the session, the caregiver is prompted to remove the tissue-applicator interface from the applicator (see Fig. 57) and clean the applicator for its subsequent use. switch to the to the next side (see Fig. 53, where the left side is selected).

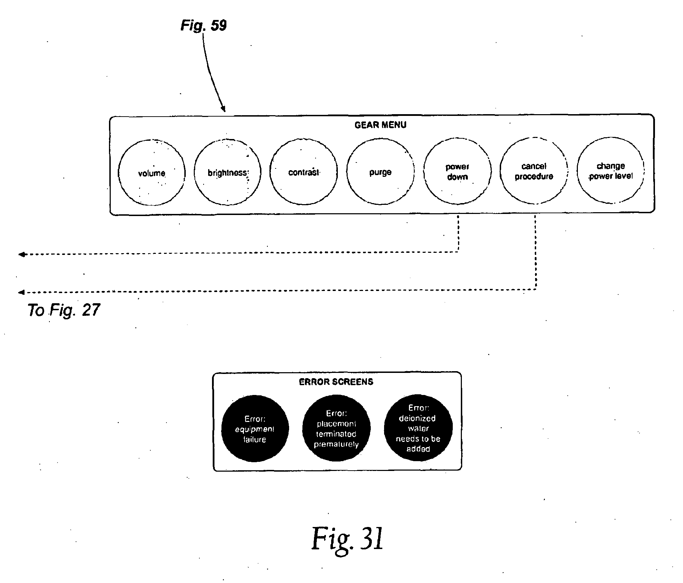

The graphical user interface 62 may also enable a gear menu (see Fig. 31). The gear menu (shown in Fig. 59) permits the caregiver to select operating conditions for the graphical user interface 62, such as, e.g., prompt volume; screen brightness and contrast, as well as certain functional operations for the console and/or applicator, such as coolant purge; power down; cancellation of a procedure; or a change in power level. The graphical user interface 62 may also enable the graphical display of error conditions (see Fig. 31), such as, e.g., equipment failure; premature termination; or low coolant levels.

Further details of the form, fit, and function of a representative graphical user interface 62 are shown in Figs. 27 to 59.

According to an embodiment of the invention, a system to apply energy to a targeted tissue region includes an applicator and a tissue-applicator interface. The applicator includes an applicator interior carrying at least one energy emitter. According to an embodiment of the invention, the tissue-applicator interface is sized and configured to be attached to the applicator for use in operative association with the energy emitter and to be detached from the applicator after use. According to an embodiment of the invention, the tissue-applicator interface comprises a bio-barrier system that, when the tissue-applicator interface is attached to the applicator, isolates the applicator interior from contact with physiologic liquids in the targeted tissue region. According to an embodiment of the invention, the bio-barrier system includes a first bio-barrier component having a prescribed conductivity to pass energy from the energy emitter to the targeted tissue region without substantial interference and loss of power.

According to an embodiment of the invention, the prescribed conductivity comprises a loss tangent tan δ of not greater than 0.1, where tan δ = σ/ωε, where σ is the conductivity of the first bio-barrier component, ω is the frequency of the energy emitted by the energy emitter, and ε is the permittivity of the first bio-barrier component.

According to an embodiment of the invention, the tissue-applicator interface includes a tissue acquisition chamber that acquires tissue in the targeted tissue region for application of energy in response to negative pressure generated by an external source and conveyed into the tissue acquisition chamber.

According to an embodiment of the invention, the bio-barrier system includes a second bio-barrier component separate from the first bio-barrier component. According to an embodiment of the invention, the second bio-barrier is substantially permeable to air to balance negative pressure between the tissue acquisition chamber and the applicator interior when the tissue-applicator interface is attached to the applicator. According to an embodiment of the invention, the second bio-barrier component is also substantially impermeable to liquids to isolate the applicator interior from contact with physiologic liquids in the targeted tissue region while balancing the negative pressure.

According to an embodiment of the invention, the first bio-barrier component is substantially impermeable to air.

According to an embodiment of the invention, the bio-barrier system includes a third bio-barrier component separate from the first and second bio-barrier components. According to an embodiment of the invention, the third bio-barrier component is substantially permeable to air to permeable to air to convey negative pressure from the source into the tissue acquisition chamber. According to an embodiment of the invention, the third bio-barrier component is also substantially impermeable to liquids to isolate the source from contact with physiologic liquids in the targeted tissue region.

According to an embodiment of the invention, the applicator includes a cooling plate, that, when the tissue-applicator interface is attached to the applicator, is sized and configured for thermal conductive contact with the first bio-barrier component. According to an embodiment of the invention, the first bio-barrier component has a prescribed thermal conductivity to allow thermal conduction to occur between the cooling plate and the targeted tissue region without substantial interference.

According to an embodiment of the invention, the prescribed thermal conductivity of the first bio-barrier component is at least 0.1 watts per meter-Kelvin (0.1 W/mK)

According to an embodiment of the invention, the applicator is sized and configured for repeated use, and the applicator is sized and configured for disposal after a single use.

According to an embodiment of the invention, the energy emitter is sized and configured to emit microwave energy.

According to an embodiment of the invention, instructions are included for using the system to treat an axilla.

According to an embodiment of the invention, a system to apply energy to a targeted tissue region includes an applicator and a console. According to an embodiment of the invention, the applicator carries at least one energy emitter and a cooling plate. The applicator includes an applicator controller communicating with the energy emitter and a sensor coupled to the cooling plate. According to an embodiment of the invention, the console includes a generator to generate a prescribed form of energy, and a cooler to cool a coolant.

According to an embodiment of the invention, the console includes a master controller including an energy generation function coupled to the generator to transmit energy to the energy emitter to form lesions in the targeted tissue region and a lesion control function coupled to the cooler to circulate coolant to the coolant plate to control lesion formation, According to an embodiment of the invention, a special purpose cable system couples the applicator to the console. According to an embodiment of the invention, the special purpose cable system includes a cable to convey energy from the generator to the energy emitter, supply and return conduits separate from the cable to circulate coolant to the cooling plate, and communication channels separate from the cable and supply and return conduits establishing a communication link between the master controller and the applicator controller.

According to an embodiment of the invention, the special purpose cable system includes a far end secured to the applicator and a near end comprising a connector sized and configured for releasable connection to a mating special purpose connection site. According to an embodiment of the invention, the mating special purpose connection site is on the console.

According to an embodiment of the invention, the prescribed form of energy comprises microwave energy.

According to an embodiment of the invention, the prescribed form of energy comprises a microwave signal that lays in the ISM band of 5.775 to 5.825 GHz, with a frequency centered at approximately 5.8 GHz.

According to an embodiment of the invention, there are included instructions for using the system to treat an axilla.

According to a disclosed example, a method to apply energy to a targeted tissue region provides a system, which is operated to form lesions in the targeted tissue region.

The method provides instructions for operating the system. The lesions are formed in an axilla.

The lesions treat hyperhidrosis.

Various features of the invention are set forth in the following claims.

## Claims

1. A system (10) to apply energy to a targeted tissue region, comprising:

    instructions for using the system (10) to treat an axilla,
    an applicator (14) including an applicator interior carrying at least one energy emitter (22),
    a tissue-applicator interface (16) sized and configured to be attached to the applicator (14) for use in operative association with the energy emitter (22) and to be detached from the applicator (14) after use, the tissue-applicator interface (16) comprising a bio-barrier system (50) that, when the tissue applicator interface is attached to the applicator, isolates the applicator interior from contact with physiologic liquids in the targeted tissue region, the bio-barrier system including a first bio-barrier component (52) having a prescribed conductivity to pass energy from the energy emitter (22) to the targeted tissue region without substantial interference and loss of power,
    wherein the energy emitter (22) is sized and configured to emit microwave energy,
    wherein the prescribed conductivity comprises a loss tangent tan $\delta$ of not greater than 0.1, where tan $\delta$ = $\sigma/\omega\epsilon$, where $\sigma$ is the conductivity of the first bio-barrier component (52), $\omega$ is the frequency of the energy emitted by the energy emitter (22), and $\epsilon$ is the permittivity of the first bio-barrier component (52),

    wherein the tissue-applicator interface (16) includes a tissue acquisition chamber (42) that acquires tissue in the targeted tissue region for application of energy in response to negative pressure generated by an external source and conveyed into the tissue acquisition chamber (42),
    wherein the bio-barrier system (50) includes a second bio-barrier component (54) separate from the first bio-barrier component (52), the second bio-barrier (54) being substantially permeable to air to balance negative pressure between the tissue acquisition chamber (42) and the applicator interior when the tissue-applicator interface (16) is attached to the applicator (14), the second bio-barrier component (54) also being substantially impermeable to liquids to isolate the applicator interior from contact with physiologic liquids in the targeted tissue region while balancing the negative pressure, and
    wherein the first bio-barrier component (52) is substantially impermeable to air.

2. A system (10) according to any preceding claim, wherein the system (10) includes a third bio-barrier component (56) separate from the first and second bio-barrier components (52, 54), the third bio-barrier component (56) being substantially permeable to air to convey negative pressure from the source into the tissue acquisition chamber (42), the third bio-barrier component (56) also being substantially impermeable to liquids to isolate the source from contact with physiologic liquids in the targeted tissue region.

3. A system (10) according to any preceding claim, wherein:

    the applicator (14) includes a cooling plate (28), that, when the tissue-applicator interface (16) is attached to the applicator (14), is sized and configured for thermal conductive contact with the first bio-barrier component (52), and
    wherein the first bio-barrier component (52) has a prescribed thermal conductivity to allow thermal conduction to occur between the cooling plate (28) and the targeted tissue region without substantial interference.

4. A system (10) according to claim 3, wherein the prescribed thermal conductivity of the first bio-barrier component (52) is at least 0.1 watts per meter-Kelvin (0.1 W/mK).

5. A system (10) according to any preceding claim, wherein:

    the applicator (14) is sized and configured for repeated use, and
    the tissue-applicator interface (16) is sized and configured for disposal after a single use.

6. A system (10) according to any of claims 1-2, wherein:

the applicator (14) carries a cooling plate (28), and includes an applicator controller (60) communicating with the energy emitter (22) and a sensor coupled to the cooling plate (28), and said system further comprises, a console (12) including a generator to generate a prescribed form of energy, and a cooler to cool a coolant, the console including a master controller (58) including an energy generation function coupled to the generator to transmit energy to the energy emitter (22) to form lesions in the targeted tissue region and a lesion control function coupled to the cooler to circulate coolant to the coolant plate (28) to control lesion formation, and a special purpose cable system (34) coupling the applicator (14) to the console (12), the special purpose cable system (34) including a cable to convey energy from the generator to the energy emitter(22), supply and return conduits (146, 150) separate from the cable to circulate coolant to the cooling plate (28), and communication channels separate from the cable and supply and return conduits establishing a communication link between the master controller (58) and the applicator controller (60).

7. A system (10) according to claim 6, wherein:

the special purpose cable system (34) includes a far end secured to the applicator (14) and a near end comprising a connector (36) sized and configured for releasable connection to a mating special purpose connection site (38), and the mating special purpose connection site (38) is on the console (12).

8. A system (10) according to claim 6 or 7, wherein the prescribed form of energy comprises microwave energy.

9. A system (10) according to any preceding claim, wherein the prescribed form of energy comprises a microwave signal that lays in the ISM band of 5.775 to 5.825 GHz, with a frequency centered at approximately 5.8 GHz.

**Patentansprüche**

1. System (10) zum Zuführen von Energie zu einer Zielgeweberegion, umfassend:

Anweisungen zur Verwendung des Systems (10) zum Behandeln einer Achsel, einen Applikator (14), der ein Applikator-Inneres umfasst, das mindestens eine Energiemissionsquelle (22) aufnimmt, ein Gewebe-Applikator-Zwischenstück (16), das so bemessen und ausgelegt ist, dass es zur Verwendung in betrieblicher Verbindung mit dem Energieemitter (22) am Applikator (14) angebracht wird und nach der Verwendung vom Applikator (14) abgenommen wird, wobei das Gewebe-Applikator-Zwischenstück (16) ein Biobarrerensystem (50) umfasst, das, wenn das Gewebe-Applikator-Zwischenstück am Applikator angebracht ist, das Applikator-Innere von Kontakt mit physiologischen Flüssigkeiten in der Zielgeweberegion isoliert, wobei das Biobarrierensystem eine erste Biobarrerenkomponente (52) mit einer vorgegebenen Leitfähigkeit umfasst, um Energie vom Energieemitter (22) ohne wesentliche Interferenz und ohne wesentlichen Leistungsverlust zur Zielgeweberegion durchzulassen, wobei der Energieemitter (22) so bemessen und ausgelegt ist, dass er Mikrowellenenergie emittiert, wobei die vorgegebene Leitfähigkeit eine Verlusttangente $\tan \delta$ von nicht mehr als 0,1 umfasst, wobei $\tan \delta = \sigma/\omega\varepsilon$, wobei $\sigma$ die Leitfähigkeit der ersten Biobarrierenkomponente (52) ist, $\omega$ die Frequenz der Energie ist, die vom Energieemitter (22) emittiert wird, und $\varepsilon$ die Permittivität der ersten Biobarrierenkomponente (52) ist, wobei das Gewebe-Applikator-Zwischenstück (16) eine Gewebeerfassungskammer (42), die Gewebe in der Zielgeweberegion zur Zufuhr von Energie in Reaktion auf Unterdruck erfasst, der durch eine externe Quelle erzeugt und in die Gewebeerfassungskammer (42) befördert wird, wobei das Biobarrierensystem (50) eine zweite Biobarrierenkomponente (54) getrennt von der ersten Biobarrierenkomponente (52) umfasst, wobei die zweite Biobarrierenkomponente (54) im Wesentlichen luftdurchlässig ist, um Unterdruck zwischen der Gewebeerfassungskammer (42) und dem Applikator-Inneren auszugleichen, wenn das Gewebe-Applikator-Zwischenstück (16) am Applikator (14) angebracht ist, wobei die zweite Biobarrierenkomponente (54) außerdem im Wesentlichen flüssigkeitsundurchlässig ist, um das Applikator-Innere von Kontakt mit physiologischen Flüssigkeiten in der Zielgeweberegion während des Ausgleichens des Unterdrucks zu isolieren, und wobei die erste Biobarrierenkomponente (52) im Wesentlichen luftundurchlässig ist.

2. System (10) nach einem der vorhergehenden Ansprüche, wobei das System (10) eine dritte Biobarrierenkomponente (56) getrennt von den ersten und zweiten Biobarnerenkomponenten (52, 54) umfasst, wobei die dritte Biobarrierenkomponente (56) im Wesentlichen luftdurchlässig ist, um Unterdruck von der Quelle in die Gewebeerfassungskammer (42) zu befördern, wobei die dritte Biobarrierenkomponente (56) außerdem im Wesentlichen flüssigkeitsundurchlässig ist, um die Quelle von Kontakt mit physiologischen Flüssigkeiten in der Zielgeweberegion zu isolieren.

3. System (10) nach einem der vorhergehenden Ansprüche, wobei:

   der Applikator (14) eine Kühlplatte (28) umfasst, die für Wärmeleitkontakt mit der ersten Biobarrierenkomponente (52), wenn das Gewebe-Applikator-Zwischenstück (16) am Applikator (14) angebracht ist, bemessen und ausgelegt ist, und wobei die erste Biobarrierenkomponente (52) eine vorgegebene Wärmeleitfähigkeit aufweist, um Wärmeleitung zwischen der Kühlplatte (28) und der Zielgeweberegion ohne wesentliche Interferenz stattfinden zu lassen.

4. System (10) nach Anspruch 3, wobei die vorgegebene Wärmeleitfähigkeit der ersten Biobarrierenkomponente (52) mindestens 0,1 Watt pro Meter und Kelvin (0,1 W/mK) beträgt.

5. System (10) nach einem der vorhergehenden Ansprüche, wobei:

   der Applikator (14) zur wiederholten Verwendung bemessen und ausgelegt ist, das Gewebe-Applikator-Zwischenstück (16) zur Entsorgung nach einer einzigen Verwendung bemessen und ausgelegt ist.

6. System (10) nach einem der Ansprüche 1 bis 2, wobei:

   der Applikator (14) eine Kühlplatte (28) trägt und eine Applikator-Steuerung (60) umfasst, die mit dem Energieemitter (22) und einem Sensor kommuniziert, der mit der Kühlplatte (28) gekoppelt ist, und das System ferner umfasst:

   eine Konsole (12), die einen Generator zum Erzeugen einer vorgegebenen Form von Energie und einen Kühler zum Kühlen eines Kühlmittels umfasst, wobei die Konsole eine Hauptsteuerung (58) umfasst, die eine Energieerzeugungsfunktion, die mit dem Generator gekoppelt ist, um Energie an den Energieemitter (22) zu übertragen, um Läsionen in der Zielgeweberegion zu bilden, und eine Läsionssteuerungsfunktion umfasst, die mit dem Kühler gekoppelt ist, um zum Steuern der Läsionsbildung Kühlmittel zur Kühlplatte (28) zirkulieren zu lassen, und

   ein Spezialkabelsystem (34), das den Applikator (14) mit der Konsole (12) koppelt, wobei das Spezialkabelsystem (34) ein Kabel zum Befördern von Energie vom Generator zum Energieemitter (22), Zu- und Rückleitungen (146, 150) getrennt vom Kabel zum Zirkulierenlassen von Kühlmittel zur Kühlplatte (28) und Kommunikationskanäle getrennt vom Kabel und den Zu- und Rückleitungen umfasst, die eine Kommunikationsverbindung zwischen der Hauptsteuerung (58) und der Applikator-Steuerung (60) herstellen.

7. System (10) nach Anspruch 6, wobei:

   das Spezialkabelsystem (34) ein fernes Ende, das am Applikator (14) befestigt ist, und ein nahes Ende umfasst, das einen Steckverbinder (36) umfasst, der zur lösbaren Verbindung mit einer passenden Spezialsteckverbinderstelle (38) bemessen und ausgelegt ist, und die passende Spezialsteckverbinderstelle (38) auf der Konsole (12) ist.

8. System (10) nach Anspruch 6 oder 7, wobei die vorgegebene Form von Energie Mikrowellenenergie umfasst.

9. System (10) nach einem der vorhergehenden Ansprüche, wobei die vorgegebene Form von Energie ein Mikrowellensignal umfasst, das im ISM-Band von 5,775 bis 5.825 GHz mit einer bei ungefähr 5,8 GHz zentrierten Frequenz liegt.

**Revendications**

1. Système (10) pour appliquer de l'énergie à une région tissulaire cible, comprenant :

   des instructions concernant l'utilisation du système (10) pour traiter une région axillaire ; un applicateur (14), englobant un intérieur d'applicateur supportant au moins un émetteur d'énergie (22) ; une interface tissus-applicateur (16), dimensionnée et configurée de sorte à être fixée sur l'applicateur (14) en vue d'une utilisation en association opérationnelle avec un émetteur d'énergie (22) et à être détachée de l'applicateur (14) après l'utilisation, l'interface tissus-applicateur (16) comprenant un système de barrière biologique (50), qui, lors de la fixation de l'interface tissus-applicateur sur l'applicateur, isole l'intérieur de l'applicateur contre un contact avec des liquides physiologiques dans la région tissulaire cible, le système de barrière biologique englobant un premier composant de barrière biologique (52) présentant une conductivité prescrite pour faire passer l'énergie de l'émetteur d'énergie (22) vers la région tissulaire cible sans interférence et perte de puissance substantielles ;

dans lequel l'émetteur d'énergie (22) est dimensionné et configuré de sorte à émettre de l'énergie micro-ondes ;

dans lequel la conductivité prescrite comprend une tangente de l'angle des pertes tan δ non supérieure à 0,1, tan δ = σ/ωε, σ représentant la conductivité du premier composant de barrière biologique (52), ω représentant la fréquence de l'énergie émise par l'émetteur d'énergie (22) et ε représentant la permittivité du premier composant de barrière biologique (52) ;

dans lequel l'interface tissus-applicateur (16) englobe une chambre d'acquisition de tissus (42), acquérant des tissus dans la région tissulaire cible en vue de l'application d'énergie en réponse à la pression négative produite par une source externe et transférée dans la chambre d'acquisition de tissus (42) ;

dans lequel le système de barrière biologique (50) englobe un deuxième composant de barrière biologique (54) séparé du premier composant de barrière biologique (52), la deuxième composant de barrière biologique (54) étant essentiellement perméable à l'air, afin d'équilibrer la pression négative entre la chambre d'acquisition des tissus (42) et l'intérieur de l'applicateur lorsque l'interface tissus-applicateur (16) est fixée sur l'applicateur (14), le deuxième composant de barrière biologique (54) étant en outre essentiellement imperméable aux liquides afin d'isoler l'intérieur de l'applicateur contre un contact avec des liquides physiologiques dans la région tissulaire cible au cours de l'équilibrage de la pression négative ; et

dans lequel le premier composant de barrière biologique (52) est essentiellement imperméable à l'air.

2. Système (10) selon l'une quelconque des revendications précédentes, dans lequel le système (10) englobe un troisième composant de barrière biologique (56), séparé des premier et deuxième composants de barrière biologique (52, 54), le troisième composant de barrière biologique (56) étant essentiellement perméable à l'air, afin de transférer la pression négative de la source dans la chambre d'acquisition des tissus (42), le troisième composant de barrière biologique (56) étant en outre essentiellement imperméable aux liquides, afin d'isoler la source contre un contact avec des liquides physiologiques dans la région tissulaire cible.

3. Système (10) selon l'une quelconque des revendications précédentes, dans lequel :

l'applicateur (14) englobe une plaque de refroidissement (28), qui est, lorsque l'interface tissus-applicateur (16) est fixée sur l'applicateur (14), dimensionnée et configurée en vue d'un contact à conduction thermique avec le premier composant de barrière biologique (52) ; et

dans lequel le premier composant de barrière biologique (52) présente une conductivité thermique prescrite pour permettre l'établissement d'une conduction thermique entre la plaque de refroidissement (28) et la région tissulaire cible, sans interférence substantielle.

4. Système (10) selon la revendication 3, dans lequel la conductivité thermique prescrite du premier composant de barrière biologique (52) correspond au moins à 0,1 Watt par mètre -Kelvin (0,1 W/mK).

5. Système (10) selon l'une quelconque des revendications précédentes, dans lequel :

l'applicateur (14) est dimensionné et configuré en vue d'une utilisation répétée ; et l'interface tissus-applicateur (16) est dimensionnée et configurée en vue d'une évacuation après une utilisation unique.

6. Système (10) selon l'une quelconque des revendications 1 à 2, dans lequel :

l'applicateur (14) supporte une plaque de refroidissement (28), et englobe un moyen de commande de l'applicateur (60) communiquant avec l'émetteur d'énergie (22), et un capteur accouplé à la plaque de refroidissement (28), ledit système comprenant en outre :

une console (12), englobant un générateur pour générer une forme d'énergie prescrite, et un moyen de refroidissement pour refroidir un liquide de refroidissement, la console englobant un moyen de commande central (58), englobant une fonction de génération d'énergie accouplée au générateur, pour transmettre l'énergie vers l'émetteur d'énergie (22) afin de former des lésions dans la région tissulaire cible, et une fonction de contrôle des lésions accouplée au moyen de refroidissement, afin de faire circuler le liquide de refroidissement vers la plaque de refroidissement (28) pour contrôler la formation des lésions ; et

un système de câble spécial (34), servant à accoupler l'applicateur (14) à la console (12), le système de câble spécial (34) englobant un câble pour transférer l'énergie du générateur vers l'émetteur d'énergie (22), des conduits d'alimentation et de retour (146, 150), séparés du câble, pour faire circuler le liquide de refroidissement vers la plaque de refroidissement (28), et des canaux de communication, séparés du câble et des conduits d'alimentation et de retour, établissant une liaison à communication entre le moyen de commande principal (58) et le moyen de commande de l'applicateur (60).

7.  Système (10) selon la revendication 6, dans lequel :

le système de câble spécial (34) englobe une extrémité éloignée fixée sur l'applicateur (14) et une extrémité proche, comprenant un connecteur (36) dimensionné et configuré en vue d'une connexion amovible à un site de connexion d'accouplement spécial (38) ; et

le site de connexion d'accouplement spécial (38) se situe sur la console (12).

8.  Système (10) selon les revendications 6 ou 7, dans lequel la forme d'énergie prescrite comprend l'énergie micro-ondes.

9.  Système (10) selon l'une quelconque des revendications précédentes, dans lequel la forme d'énergie prescrite comprend un signal micro-ondes se situant dans la bande ISM de 5,775 à 5,825 GHz, avec une fréquence centrée à environ 5,8 GHz.

*Fig. 1*

*Fig. 2*

*Fig. 3*

Fig. 4

Fig. 5

Fig. 6

Fig. 7

Fig. 8

Fig. 9

Fig. 10

Fig. 11

Fig. 12A

Fig. 12B

22

24

78

80

42    28

92

*Fig. 13*

106

50

50

44

92

*Fig. 14A*
(Looking Toward Tissue)

114

42

92

*Fig. 14B*
(Looking Toward Applicator)

114

Fig. 15

Fig. 16A

Fig. 16B

Fig. 16C

Fig. 16D

Forward Power Detecter

Forward Signal Conditioning

Forward Power Attenuator/DC Block

Directional Coupler

Supply In
(From Applicator Main Board)

DC Out Forward
(To Applicator Main Board)

Reverse Signal Conditioning

DC Out Reverse
(To Applicator Main Board)

Reverse Power Attenuator/DC Block

Reverse Power Detecter

Fig. 17

Fig. 18

EP 2 349 167 B1

Fig. 19A

Backlight
Ready - Green
Operating - Blue
Fault - Red

174

30

14

Fig. 19B

Initiation
See Fig. 47

Fig. 19C

Treatment
Ending
See Fig. 49

Treatment
in Progress
See Fig. 48

Fig. 19D

Fig. 19E

Fig. 20

Fig. 21A

*Fig. 21B*

*24*

*100*

*A*  *B*  *C*  *D*

*28*

*42*

*16*

Epidermis
Dermis
Interface

Lesion Region 1   Lesion Region 3
Lesion Region 2   Lesion Region 4

Sweat
Gland   Muscle   Hypodermis

*Fig. 22A*

Tissue

Lesion

*B*

*Fig. 22B*

*Fig. 23A*

*Fig. 23B*

Fig. 24A

Fig. 24B

*Fig. 25*

Fig. 26

Fig. 27

If user does not comply within X ... down?

after X seconds, an indicator tells user what to do

indication of invalid scan appears briefly

*Fig. 37*

N

N

user touches "start procedure" icon

illustration instructs user to scan disposable

scan detected?

Y

correct scan?

Y

positive confirmation of scan appears

*From Fig. 27*

*Fig. 36*

advances to next screen automatically

*Fig. 39*

install disposable to wand and generator

choose touch up mode or regular visit

*To Fig. 29*

*Fig. 38*

touch-up mode

**TOUCH-UP MODE - NO PITS TREATED**

use touch-up template to apply marker dots

touchup anaesthesia

touch-up treatment

invoke antennae drop-down

touch desired setting

*Fig. 58*

change number of antennae

tap OK button

*To Fig. 27*

*Fig. 28*

45

Fig. 40    Fig. 41    Fig. 42    Figs. 43, 44    Fig. 45    Fig. 46    Fig. 47

regular
visit

template
height and
width

apply
transfer

aneasthesia
intro
showing
"specs"

instruct right
side = 1, left
side = 2

inject right

inject left

instruct to
start on the
right side

From
Fig. 28

tap OK button

tap "wrench"

To
Fig. 30

option to
change
starting
side

Fig. 29

46

*Fig. 30*

*Fig. 59*

**GEAR MENU**

volume    brightness    contrast    purge    power down    cancel procedure    change power level

*To Fig. 27*

**ERROR SCREENS**

Error: equipment failure    Error: placement terminated prematurely    Error: deionized water needs to be added

*Fig. 31*

*Fig. 32*

**Testing...**

*Fig. 33*

Note: Exact docking mechanism unclear from photos of model, this is a placeholder only

Fig. 34

Fig. 35

Fig. 36

Fig. 37

*Fig. 38*

*Fig. 39*

Fig. 40

Fig. 41

Fig. 42

Fig. 43

Fig. 44

Fig. 45

Fig. 46

Fig. 47

Fig. 48

Fig. 49

Fig. 50

Fig. 51

Fig. 52

Fig. 53

Fig. 54

Fig. 55

Fig. 56

Fig. 57

Fig. 58

Fig. 59

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20010050083 A, Marchitto
- US 5097846 A, Larsen
- US 20070179482 A, Anderson